(19) Europäisches Patentamt
European Patent Office
Office européen des brevets

(11) EP 0 808 303 B1

(12) EUROPEAN PATENT SPECIFICATION

(45) Date of publication and mention of the grant of the patent:
**20.06.2001 Bulletin 2001/25**

(21) Application number: **96901904.1**

(22) Date of filing: **08.02.1996**

(51) Int Cl.$^7$: **C07D 211/52**, C07D 211/58, C07D 401/06, C07D 409/12, A61K 31/445, C07D 401/12, C07D 405/12, C07D 401/04, C07D 471/10

(86) International application number:
**PCT/GB96/00259**

(87) International publication number:
**WO 96/24582 (15.08.1996 Gazette 1996/37)**

# (54) 5-(4-SUBST.-PIPERIDINYL-1)-3-ARYL-PENTANOIC ACID DERIVATIVES AS TACHYKININ RECEPTOR ANTAGONIST

5-(4-SUBST.-PIPERIDINYL-1)-3-ARYL-PENTANSÄURE DERIVATE ALS TACHYKININ REZEPTOR ANTAGONISTEN

DERIVES D'ACIDE 5-(4-SUBSTITUE-PIPERIDINYL-1)-3-ARYL-PENTANOIQUE UTILISES COMME ANTAGONISTES DES RECEPTEURS DE LA TACHYKININE

(84) Designated Contracting States:
**AT BE CH DE DK ES FR GB GR IE IT LI LU MC NL PT SE**

(30) Priority: **10.02.1995 GB 9502644**

(43) Date of publication of application:
**26.11.1997 Bulletin 1997/48**

(73) Proprietor: **AstraZeneca AB**
**151 85 Södertälje (SE)**

(72) Inventors:
- **BERNSTEIN, Peter, Robert**
  **Wilmington, DE 19897 (US)**
- **DEMBOFSKY, Bruce, Thomas**
  **Wilmington, DE 19897 (US)**
- **JACOBS, Robert, Toms**
  **Wilmington, DE 19897 (US)**

(74) Representative: **Bill, Kevin et al**
**ZENECA Pharmaceuticals**
**Intellectual Property Department**
**Mereside**
**Alderley Park**
**Macclesfield, Cheshire SK10 4TG (GB)**

(56) References cited:
**EP-A- 0 428 434**
**WO-A-94/10146**
**WO-A-94/29309**
**FR-A- 2 100 711**
**US-A- 5 434 158**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 808 303 B1

## Description

This invention concerns novel substituted 5-(heterocyclic)valeryl derivatives which antagonize the pharmacological actions of the endogenous neuropeptide tachykinins known as neurokinins, particularly at the neurokinin 1 (NK1) and the neurokinin 2 (NK2) receptors. The novel 5-(heterocyclic)valeryl derivatives are useful whenever such antagonism is desired. Thus, such compounds may be of value in the treatment of those diseases in which the NK1 and/or NK2 receptor is implicated, for example, in the treatment of asthma and related conditions. The invention also provides pharmaceutical compositions containing the novel 5-(heterocyclic)valeryl derivatives for use in such treatment, methods for their use, and processes and intermediates for the manufacture of the novel 5-(heterocyclic)valeryl derivatives.

The mammalian neurokinins comprise a class of peptide neurotransmitters which are found in the peripheral and central nervous systems. The three principal neurokinins are SP (SP), Neurokinin A (NKA) and Neurokinin B (NKB). There are also N-terminally extended forms of at least NKA. At least three receptor types are known for the three principal neurokinins. Based upon their relative selectivities favoring the neurokinin agonists SP, NKA and NKB, the receptors are classifed as neurokinin 1 (NK1), neurokinin 2 (NK2) and neurokinin 3 (NK3) receptors, respectively. In the periphery, SP and NKA are localized in C-afferent sensory neurons, which neurons are characterized by non-myelinated nerve endings known as C-fibers, and are released by selective depolarization of these neurons, or selective stimulation of the C-fibers. C-Fibers are located in the airway epithelium, and the tachykinins are known to cause profound effects which clearly parallel many of the symptoms observed in asthmatics. The effects of release or introduction of tachykinins in mammalian airways include bronchoconstriction, increased microvascular permeability, vasodilation, increased mucus secretion and activation of mast cells. Thus, the tachykinins are implicated in the pathophysiology and airway hyperresponsiveness observed in asthmatics; and blockade of the action of released tachykinins may be useful in the treatment of asthma and related conditions. A cyclopeptide antagonists (FK-224) selective for both NK1 and NK2 receptors has demonstrated clinical efficacy in human patients suffering from asthma and chronic bronchitis. M. Ichinose, et al., Lancet, 1992, 340, 1248. Nonpeptidic tachykinin antagonists have been reported, for example in European Patent Application, Publication Number (EPA) 428434, EPA 474561, EPA 512901, EPA 512902, EPA 515240 and EPA 559538, as well as in WO 94/10146, EPA 0625509, EPA 0630887, WO 95/05377, WO 95/12577, WO 95/15961, EPA 680962, and WO 95/16682. US 5,434,158, which published after the priority date of the present invention, and WO 94/29309 disclose certain azacycles having neurokinin antagonist activity. FR-A-2100711 discloses a series of substituted piperidine compounds having anti-diarrhoeal and analgesic activity. We have discovered a series of non-peptidic antagonists of the NK1 and NK2 receptors, and this is the basis for our invention.

According to the invention, there is provided a Compound of the invention which is a compound of formula I

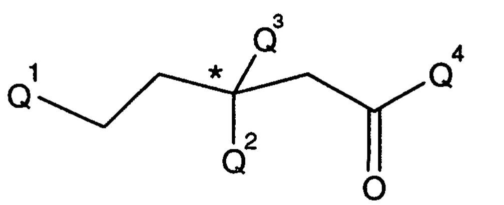

I

wherein

$Q^1$ is 4-hydroxy-4-phenylpiperidino or 4-acetamido-4-phenylpiperidino;

$Q^2$ is phenyl which may bear one or two substituents independently selected from halo, trifluoromethyl, hydroxy, (1-3C)alkoxy, (1-3C)alkyl and methylenedioxy; or $Q^2$ is thienyl, imidazolyl, benzo[b]thiophenyl or naphthyl any of which may bear a halo substituent; or $Q^2$ is biphenylyl; or $Q^2$ is carbon-linked indolyl which may bear a benzyl substituent at the 1-position; $Q^3$ is hydrogen, or (1-4C)alkyl; and

$Q^4$ is $-OR^2$ or $-NR^3R^4$; wherein

$R^2$ is hydrogen, (1-6C)alkyl, (3-7C)cycloalkyl, aryl(1-3C)alkyl or heteroaryl(1-3C)alkyl, wherein an aryl or heteroaryl group may bear one, two or three substituents independently selected from halo, trifluoromethyl, hydroxy, (1-3C) alkoxy, (1-3C)alkyl, cyano, $-S(=O)_2NR^5R^6$, $-NR^7R^8$, $C(=O)NR^9R^{10}$, and methylenedioxy, and further wherein any arylethyl, arylpropyl, heteroarylethyl or heteroarylpropyl group may optionally be substituted at the position $\alpha$ to the aryl or heteroaryl group by a group selected from oxo, and $=NOR^{11}$;

$R^3$ and $R^4$ are independently selected from hydrogen, (1-6C)alkyl, (3-7C)cycloalkyl, aryl, heteroaryl, aryl(1-3C) alkyl and heteroaryl(1-3C)alkyl, wherein any aryl or heteroaryl group may bear one two or three substituents independently selected from halo, trifluoromethyl, hydroxy, (1-3C)alkoxy, (1-3C)alkyl, cyano, $-S(=O)_2NR^5R^6$,

$-NR^7R^8$, $C(=O)NR^9R^{10}$, and methylenedioxy, and further wherein any arylethyl, arylpropyl, heteroarylethyl or heteroarylpropyl group may optionally be substituted at the position α to the aryl or heteroaryl group by a group selected from oxo, and $=NOR^{11}$; or

$-NR^3R^4$ taken together represents a cyclic amino radical selected from pyrrolidinyl, piperidino, 1,2,3,6-tetrahydropyridyl, 1,2,3,4-tetrahydroquinolyl, and 1,2,3,4-tetrahydroisoquinolyl, which cyclic amino radical may bear one or two substituents independently selected from halo, trifluoromethyl, hydroxy, (1-3C)alkoxy, (1-3C)alkyl, cyano, $-S(=O)^2NR^5R^6$, $-NR^7R^8$, $C(=O)NR^9R^{10}$, phenyl, acetamidomethyl, and methylenedioxy; and

$R^5$-$R^{11}$ are independently selected from hydrogen and (1-3C)alkyl; and wherein the term aryl denotes a phenyl radical or an ortho-fused bicyclic carbocyclic radical having nine to ten ring atoms in which at least one ring is aromatic and the term heteroaryl means a radical of a monocyclic aromatic ring containing five ring atoms, consisting of carbon and one to four heteroatoms selected from oxygen, sulfur and nitrogen or containing six ring atoms consisting of carbon and one or two nitrogens, as well as a radical of an ortho-fused bicyclic heterocycle of eight to ten atoms derived therefrom, or a stable N-oxide thereof;

or the N-oxide of the piperidino nitrogen in $Q^1$;

or a pharmaceutically acceptable salt thereof;

or a quaternary ammonium salt thereof in which the piperidino nitrogen in $Q^1$ is a quadricovalent ammonium nitrogen wherein the fourth radical on the nitrogen $R^1$ is (1-4C)alkyl or benzyl and the associated counterion A is a pharmaceutically acceptable anion.

Another particular sub-set of compounds of the invention are compounds of formula I wherein:

$Q^1$ is 4-hydroxy-4-phenylpiperidino or 4-acetamido-4-phenylpiperidino;

$Q^2$ is phenyl which may bear one or two substituents independently selected from halo, trifluoromethyl, hydroxy, (1-3C)alkoxy, (1-3C)alkyl and methylenedioxy; or $Q^2$ is thienyl, imidazolyl, benzo[b]thiophenyl or naphthyl any of which may bear a halo substituent; or $Q^2$ is biphenylyl; or $Q^2$ is carbon-linked indolyl which may bear a benzyl substituent at the 1-position;

$Q^3$ is hydrogen; and

$Q^4$ is $-OR^2$ or $-NR^3R^4$; wherein

$R^2$ is hydrogen, (1-6C)alkyl, (3-7C)cycloalkyl, aryl(1-3C)alkyl or heteroaryl(1-3C)alkyl, wherein an aryl or heteroaryl group may bear one, two or three substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, and methylenedioxy;

$R^3$ and $R^4$ are independently selected from hydrogen, (1-6C)alkyl, (3-7C)cycloalkyl, aryl, heteroaryl, aryl(1-3C)alkyl and heteroaryl(1-3C)alkyl, wherein any aryl or heteroaryl group may bear one two or three substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, and methylenedioxy; or

the group $-NR^3R^4$ taken together represents a cyclic amino radical selected from pyrrolidinyl, piperidino, 1,2,3,6-tetrahydro-pyridyl, 1,2,3,4-tetrahydroquinolyl, and 1,2,3,4-tetrahydroisoquinolyl, which cyclic amino radical may bear one or two substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, phenyl, acetamidomethyl, and methylenedioxy; and

$R^5$-$R^{11}$ are independently selected from hydrogen and (1-3C)alkyl;

or the N-oxide of the nitrogen in $Q^1$;

or a pharmaceutically acceptable salt thereof;

or a quaternary ammonium salt thereof in which the nitrogen in $Q^1$ is a quadricovalent ammonium nitrogen wherein the fourth radical on the nitrogen $R^1$ is (1-4C)alkyl or benzyl and the associated counterion A is a pharmaceutically acceptable anion.

It will be appreciated that a compound of formula I may contain one or more asymmetically substituted carbon atoms and that such a compound may be isolated in optically active, racemic and/or diastereomeric forms. A compound may exhibit polymorphism. It is to be understood that the present invention encompasses any racemic, optically-active, diastereomeric, polymorphic or stereoisomeric form, or mixture thereof, which form possesses NK1 and NK2 antagonist properties, it being well known in the art how to prepare optically-active forms (for example, by resolution of the racemic form or by synthesis from optically-active starting materials) and how to determine the NK1 and NK2 antagonist properties by the standard tests known in the art and those described hereinafter. It may be preferred to use the compound of formula I in a form which is characterized as containing, for example, at least 95%, 98% or 99% enantiomeric excess of the form which is of the (S)-configuration at the center indicated by * in formula I.

In this specification $R^1$, $R^2$, et cetera stand for generic radicals and have no other significance. It is to be understood that the generic terms "(1-3C)alkyl" and "(1-6C)alkyl" include both straight and branched chain alkyl radicals but references to individual alkyl radicals such as "propyl" embrace only the straight chain ("normal") radical, branched

chain isomers such as "isopropyl" being referred to specifically. A similar convention applies to other generic groups, for example, alkoxy, et cetera. Halo is fluoro, chloro, bromo or iodo. Aryl denotes a phenyl radical or an ortho-fused bicyclic carbocyclic radical having about nine to ten ring atoms in which at least one ring is aromatic. Heteroaryl encompasses a radical of a monocyclic aromatic ring containing five ring atoms, consisting of carbon and one to four heteroatoms selected from oxygen, sulfur and nitrogen or containing six ring atoms consisting of carbon and one or two nitrogens, as well as a radical of an ortho-fused bicyclic heterocycle of about eight to ten atoms derived therefrom, particularly a benz-derivative or one derived by fusing a propenylene, trimethylene of tetramethylene diradical thereto, as well as a stable N-oxide thereof.

Particular values listed below for radicals, substituents and ranges are for illustration only and they do not exclude other defined values or other values within defined ranges for the radicals and substituents.

A particular value for aryl is phenyl. A particular value for heteroaryl is furyl, pyridyl, imidazolyl, indolyl or pyrimidinyl. A particular value for halo is chloro or bromo. A particular value for (1-3C)alkyl is methyl, ethyl, propyl or isopropyl; for (1-4C)alkyl is methyl, ethyl, propyl, isopropyl, butyl, isobutyl or t-butyl; for (1-5C)alkyl is methyl, ethyl, propyl, isopropyl, butyl, isobutyl, t-butyl, pentyl or isopentyl; and for (1-6C)alkyl is methyl, ethyl, propyl, isopropyl, butyl, isobutyl, t-butyl, pentyl, isopentyl, hexyl or isohexyl. A particular value for (3-7C)cycloalkyl is cyclopropyl, cyclopentyl, cyclohexyl or cycloheptyl.

A more particular value for heteroaryl is pyridyl. A more particular value for halo is chloro. A more particular value for (1-3C)alkyl is methyl; for (1-4C)alkyl is methyl or ethyl; for (1-5C)alkyl is methyl, ethyl, propyl or isopropyl; and for (1-6C)alkyl is methyl, ethyl, propyl, isopropyl, butyl, isobutyl or t-butyl. A more particular value for (3-7C)cycloalkyl is cyclopropyl or cyclopentyl.

A particular value for $Q^2$ 3,4-dichlorophenyl, or 3,4-methylenedioxyphenyl; for $Q^3$ is hydrogen; and for $Q^4$ is benzylamino, 4-phenylpiperidino, 4-methoxybenzylamino, cyclohexylamino, 4-methylbenzylamino, (benzyl)(methyl) amino, (methyl)(phenyl)amino, phenylamino, benzyloxy, (2-methoxybenzyl)-(methyl)amino, [3,5-bis(trifluoromethyl) benzyl](methyl)amino, 2-methoxybenzylamino, ethoxy, (3,5-dichloro-2-methoxybenzyl)amino, N-[3,5-dichloro-2-methoxybenzyl]-N-methylamino or 3,5-bis(trifluoromethyl)benzylamino.

A more particular value for $Q^2$ is 3,4-dichlorophenyl; and for $Q^4$ is (2-methoxybenzyl)(methyl)amino.

A particular group of compounds of formula I are compounds of formula III

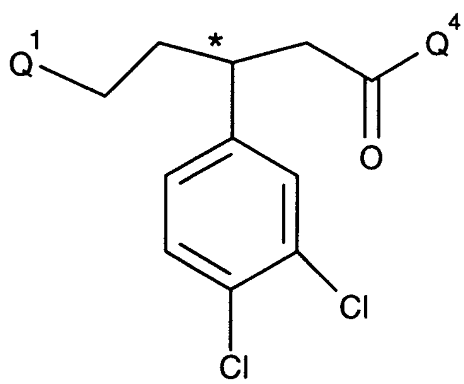

III

wherein,

$Q^1$ and $Q^4$ have any of the values defined above.

A particular group of compounds of formula I are compounds wherein $Q^4$ is $-OR^2$.

A particular group of compounds of formula I are compounds wherein $Q^4$ is $NR^3R^4$.

A more particular group of compounds of formula I are compounds of formula III wherein, $Q^4$ is $-OR^2$.

A more particular group of compounds of formula I are compounds of formula III wherein, $Q^4$ is $NR^3R^4$.

Pharmaceutically acceptable salts of a compound of formula I include those made with a strong inorganic or organic acid which affords a physiologically acceptable anion, such as, for example, hydrochloric, sulfuric, phosphoric, methanesulfonic, or para-toluenesulfonic acid.

A compound of formula I may be made by processes which include processes known in the chemical art for the production of structurally analogous heterocyclic compounds. Such processes and intermediates for the manufacture of a compound of formula I as defined above are provided as further features of the invention and are illustrated by the following procedures in which the meanings of generic radicals are as defined above unless otherwise indicated:

(a) Acylating an amine of formula $-NR^3R^4$, with an ester of formula IV

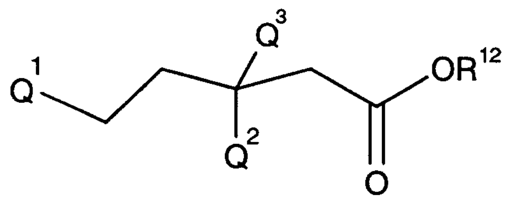

IV

wherein $R^{12}$ is a suitable alkyl radical such as for example (1-3C)alkyl. The acylation may conveniently be carried out in a suitable solvent, such as for example diethyl ether, benzene or toluene, at a suitable temperature such as for example a temperature in the range of -50 to 100 °C preferably in the range of 0 to 60 °C. The reaction may conveniently be carried out in the presence of a trialkyl aluminum compound such as trimethylaluminum. Suitable conditions for the acylation are described in Example 1.

(b) For an acid addition salt of a compound of formula I, treating a corresponding compound of formula I which is in the free-base form, with an acid. The salt may conveniently be formed in a suitable solvent, such as for example diethyl ether, benzene or toluene. Suitable conditions for the formation of an acid-addition salt of a compound of formula I can be found at Example 2.

(c) Alkylating an amine of formula $Q^1$-H with an aldehyde of formula V

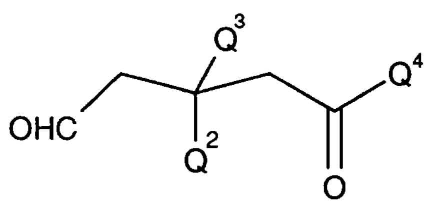

V

by reductive alkylation. The alkylation may conveniently be carried out under conventional reductive alkylation conditions, for example by the in situ acid-catalyzed formation of an imminium salt, followed by reduction with sodium cyanoborohydride in alcoholic solvent. The reaction may be carried out in a suitable solvent at a temperature in the range of -20 to 100 °C, preferably in the range of 0 to 50 °C. Suitable conditions for the alkylation I can be found at Example 9.

(d) Acylating an amine of formula $-NR^3R^4$, with an acid of formula IV wherein $R^{12}$ is a hydrogen. The acylation may conveniently be carried out in a suitable solvent, such as for example N,N-dimethylformamide or tetrahydrofuran, at a suitable temperature such as for example a temperature in the range of -50 to 100 °C preferably in the range of 0 to 60 °C in the presence of a conventional coupling reagent. Suitable conditions for the acylation are described in Example 10.

(e) Alkylating an amine of formula $Q^1$-H with an alkylating agent of formula VI

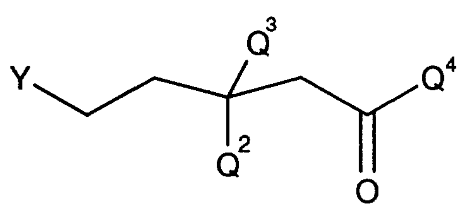

VI

in which Y is a conventional leaving group such as for example iodide, bromide, methanesulfonate, p-toluenesulfonate, or trifluoromethanesulfonate. The reaction may be carried out under standard conditions for example in a suitable solvent at a temperature in the range of -20 to 100 °C, preferably in the range of 0 to 50 °C.

(f) For an N-oxide of the nitrogen in $Q^1$ oxidizing the nitrogen of a corresponding compound of formula I using a conventional procedure, such as, for example, using hydrogen peroxide in methanol, peracetic acid, 3-chloroperoxybenzoic acid in an inert solvent (such as dichloromethane) or dioxirane in acetone.

(g) For a quaternary ammonium salt of the nitrogen in $Q^1$ alkylating the nitrogen in a corresponding compound

of formula I with an alkylating agent of formula $R^1Y$ wherein Y is a leaving group.

(j) For a compound of formula I which bears an aromatic hydroxy group, cleaving the ether of a corresponding compound of formula I which bears an aromatic alkoxy group using a conventional method.

It may be desired to optionally use a protecting group during all or portions of the above described processes; the protecting group then may be removed when the final compound is to be formed.

Whereafter, for any of the above procedures, when a pharmaceutically acceptable salt of a compound of formula I is required, it may be obtained by reacting the compound of formula I with an acid affording a physiologically acceptable counterion or by any other conventional procedure.

It will also be appreciated that certain of the various optional substituents in the compounds of the invention may be introduced by standard aromatic substitution reactions or generated by conventional functional group modifications either prior to or immediately following the processes above, and as such are included in the process aspect of the invention. The reagents and reaction conditions for such procedures are well known in the chemical art.

If not commercially available, the necessary starting materials for the above procedures may be made by procedures which are selected from standard techniques of organic chemistry, techniques which are analogous to the synthesis of known, structurally similar compounds and techniques which are analogous to the above described procedures or the procedures described in the Examples. The starting materials and the procedures for their preparation are additional aspects of the invention.

Piperidines of formula $Q^1$-H can be prepared from readily available starting materials using known synthetic methods. For example, preparations of piperidines of formula $Q^1$-H are disclosed in European Patent Applications, Publication Numbers (EPA) 428434, EPA 474561, EPA 512901, EPA 512902, EPA 515240 and EPA 559538, as well as in WO 94/10146, EPA 0625509 and EPA 0630887, WO 95/05377, WO 95/12577, WO 95/15961, EPA 680962, and WO 95/16682.. As will be clear to one skilled in the art, a variety of sequences are available for preparation of the starting materials, and the sequences leading to the starting materials and products of the invention may be altered if appropriate considerations regarding the synthetic methods and radicals present are followed.

The utility of a compound of the invention or a pharmaceutically acceptable salt thereof (hereinafter, collectively referred to as a "Compound") may be demonstrated by standard tests and clinical studies, including those disclosed in the EPA publications noted above, and those described below.

SP Receptor Binding Assay (Test A)

The ability of a Compound of the invention to antagonize the binding of SP at the NK1 receptor may be demonstrated using an assay using the human NK1 receptor expressed in Mouse Erythroleukemia (MEL) cells. The human NK1 receptor was isolated and characterized as described in: B. Hopkins, et al. "Isolation and characterization of the human lung NK1 receptor cDNA" Biochem. Biophys. Res. Comm., 1991, 180, 1110-1117; and the NK1 receptor was expressed in Mouse Erythroleukemia (MEL) cells using a procedure similar to that described in Test B below.

Neurokinin A (NKA) Receptor Binding Assay (Test B)

The ability of a Compound of the invention to antagonize the binding of NKA at the NK2 receptor may be demonstrated using an assay using the human NK2 receptor expressed in Mouse Erythroleukemia (MEL) cells, as described in: Aharony, D., et al. "Isolation and Pharmacological Characterization of a Hampster Neurokinin A Receptor cDNA" Molecular Pharmacology, 1994, 45, 9-19. In an initial use of this assay, the $IC_{50}$ measured for the standard compound L-659,877 was found to be 30 nM versus $^3$H-NKA binding to MELM.

The selectivity of a Compound for binding at the NK1 and the NK2 receptors may be shown by determining its binding at other receptors using standard assays, for example, one using a tritiated derivative of NKB in a tissue preparation selective for NK3 receptors. In general, the Compounds of the invention demonstrated statistically significant binding activity in Test A and Test B. Compounds having at least 25% inhibition at 1 µm were subject to Ki determination. For example, the compound of Example 1 demonstrated a Ki of 30 nanomolar in Test A, and a Ki of 15 nanomolar in Test B.

Rabbit Pulmonary Artery: NK1 in vitro functional assay (Test C)

The ability of a Compound of the invention to antagonize the action of the agonist, Ac-[$Arg^6$, $Sar^9$, $Met(O_2)^{11}$] SP(6-11) (designated ASMSP) in a pulmonary tissue may be demonstrated using a functional assay which is carried out under conditions similar to those described in: Emonds-Alt, X., et al. "In vitro and in vivo biological activities of Sr 140333, a novel potent non-peptide tachykinin $NK_1$ receptor antagonist" Eur. J. Pharmacol., 1993, 250, 403-413; and which is carried out as follows.

Male New Zealand white rabbits are killed by lethal injection (Nembutal, 60 mg/kg into a cannulated ear vein).

Heparin, 0.0025 ml/kg of a 1000 U/ml solution, is injected into the ear vein prior to nembutal in order to decrease blood coagulation. The left and right branches of the pulmonary artery are isolated from the rest of the lung tissue and cut in half to provide four ring segments from each animal. The segments, with intact endothelium, are suspended between stainless steel stirrups and placed in water-jacketed (37.0°C) tissue baths containing physiological salt solution of the following composition (mM): NaCl, 119.0; KC1 4.6; $CaCl_2$, 1.8; $MgCl_2$, 0.5; $NaH_2PO_4$, 1.0; $NaHCO_3$, 25.0; glucose 11.0; indomethacin 0.005 (to inhibit cyclooxygenase); and dl-propranolol, 0.001 (to inhibit b-adrenergic receptors); gassed continuously with 95% $O_2$-5% $CO_2$. Initial tension placed on each tissue is 2 grams, which is maintained throughout a 0.5 hour equilibration period. Changes in tension are measured on a Grass polygraph via Grass FT-03 force transducers.

Thiorphan, 1 X $10^{-6}$M (to inhibit E.C.3.4.24.11), and a selective NK2 antagonist (to inhibit $NK_2$ receptors) such as for example, an antagonist described in WO 94/148,184, EPA 0625509, EPA 0630887, or the antgonist SR48968 (3 X $10^{-8}$M), are added to the tissue baths along with the test compound or its vehicle 90 minutes before the $NK_1$ receptor agonist, Ac-[$Arg^6$, $Sar^9$, $Met(O_2)^{11}$]SP(6-11) (designated ASMSP). Phenylephrine, 3 X $10^{-6}$M, is added in order to induce tone in the tissue. One hour after introducing phenylephrine, cumulative concentration response effects of ASMSP are obtained and papaverine, 1 X $10^{-3}$M, is added at the end of each experiment to determine the maximum magnitude of relaxation (defined as 100%).

Potencies of the compounds are determined by calculating the apparent dissociation constants ($K_B$) for each concentration tested using the standard equation:

$$K_B = [\text{antagonist}]/(\text{dose ratio} - 1)$$

where dose ratio = antilog[(agonist-log molar $EC_{50}$ without compound) - (agonist-log molar $EC_{50}$ with compound)]. The $K_B$ values are converted to the negative logarithms and expressed as -log molar $K_B$ (i.e. $pK_B$). The potency of the agonist is determined at 50% of its own maximum relaxation in each curve. The $EC_{50}$ values are converted to negative logarithms and expressed as -log molar $EC_{50}$. Maximum relaxation responses to ASMSP are determined by expressing the maximum response to the agonist as a percentage of the relaxation caused by papaverine.

Guinea Pig Trachea Assay: NK2 in vitro functional assay (Test D)

The ability of a Compound of the invention to antagonize the action of the agonist, [b-Ala8]-Neurokinin A(4-10) (designated BANK), in a pulmonary tissue may be demonstrated using a functional assay in guinea pig trachea which is carried out under conditions similar to those described in: Ellis, J.L., et al., "Pharmacological examination of receptors mediating contractile responses to tachykinins in airways isolated from human, guinea pig and hamster" J. Pharmacol. Exp. Ther., 1993, 267, 95-101; and which is carried out as follows.

Male guinea pigs are killed by a sharp blow to the back of the head followed by exsanguination. The trachea are removed, trimmed of excess tissue (including removal of epithelium) and cut in spiral fashion. Each longitudinally cut tracheal segment is suspended as a strip in a water-jacketed (37.5°C) tissue bath containing a physiological salt solution of the following composition (mM): NaCl, 119; KCl 4.6; $CaCl_2$, 1.8; $MgCl_2$, 0.5; $NaH_2PO_4$, 1; $NaHCO_3$, 25; glucose, 11; and indomethacin, 0.005 (to inhibit cyclooxygenase); gassed continuously with 95% O2-%5 $CO_2$. Initial tension placed on each tissue is 5 g, which is maintained throughout a 0.5 hour equilibration period before addition of other drugs. Contractile responses are measured on a Grass polygraph via Grass FT-03 force transducers.

Tissues are challenged once with a single concentration of capsaicin (1 X $10^{-6}$M) and washed extensively before addition of a selective NK1 antagonist, such as for example (±)-CP96345 (3 X $10^{-7}$M) (to block NK1 receptors) and thiorphan, 1 X $10^{-6}$M (to block E.C.3.4.24.11). Cumulative addition of the $NK_2$ agonist [β-Ala8]-Neurokinin A(4-10) (designated BANK) is begun 35 minutes after addition of thiorphan. Test compound is added 120 min before BANK.

Potencies of the compounds are evaluated by calculating apparent dissociation constants ($K_B$) for each concentration tested using the standard equation:

$$K_B = [\text{antagonist}]/(\text{dose ratio}-1)$$

where dose ratio = antilog[(agonist -log molar $EC_{50}$ without compound) - (agonist-log molar $EC_{50}$ with compound)]. The $K_B$ values are converted to the negative logarithms and expressed as -log molar $K_B$ (i.e. $pK_B$). The potency of BANK is determined at 50% of its own maximum response level in each curve. The $EC_{50}$ values are converted to the negative logarithms and expressed as -log molar $EC_{50}$. Maximum contractile responses to BANK are determined by expressing the maximum response to BANK as a percentage of the initial contraction caused by capasacin.

In general, the Compounds of the invention which were tested demonstrated functional activity in Tests C and D, with a pKB of 5 or greater typically being measured in each test. For Example, the compound of Example 1 demonstrated a pKB of 5.41 in Test C, and a pKB of 6.10 in Test D.

Guinea Pig Labored Abdominal Breathing (Dyspnea) Assay: $NK_1$ and $NK_2$ in vivo functional assay (Test E)

The activity of a compound as an antagonist of $NK_1$ or $NK_2$ receptors also may be demonstrated in vivo in laboratory animals, for example by adapting a routine guinea pig aerosol test described for evaluation of leukotriene antagonists in: Snyder, et al. "Conscious guinea-pig aerosol model for evaluation of peptide leukotriene antagonists" J. Pharmacol. Meth., 1988, 19, 219, which is carried out as follows.

Using the clear plastic chamber described previously by Snyder et al. to secure guinea pigs for a head-only aerosol exposure to bronchoconstrictor agonists, agonist is administered by aerosol to six conscious guinea pigs simultaneously during each maneuver. The tachykinin $NK_1$-selective agonist ASMSP or the tachykinin $NK_2$-selective agonist, BANK, 3 X $10^{-5}$M of either, is aerosolized from a Devilbiss Model 25 ultrasonic nebulizer into an air stream entering the chamber at a rate of 2 L/minute.

Guinea pigs (275 - 400 g) are fasted for approximately 16 hours prior to experimentation. Compounds to be evaluated for blockade of effects of ASMSP or BANK or their vehicle (10% PEG400 in saline) are given by p.o., i.v. or aerosol routes of administration at various times before aerosol agonist challenge. All animals are pretreated with atropine (10 mg/kg, i.p., 45 minutes pretreatment) indomethacin (10 mg/kg, i.p. 30 minutes pretreatment), propranolol (5 mg/kg, i.p., 30 minutes pretreatment), and thiorphan (1 mg/ml aerosol for 5 minutes, 15 minutes pretreatment).

Aerosol challenge with the agonist produces an initial increase in respiratory rate followed by a decrease with early signs of minor involvement of the abdominal muscles. The respiratory rate decreases further and the breathing becomes more labored with greater involvement of the abdominal muscles as exposure continues. The distinctly recognizable end point is the point where the breathing pattern of the guinea pig is consistently slow, deep, and deliberate, showing marked involvement of the abdominal muscles. Time, in seconds, from the onset of aerosol challenge to this end point is determined for each animal by using a stopwatch. The animals generally collapsed after reaching the end point and did not recover from the agonist-induced respiratory distress. Antagonists result in an increase in the time to reach the end point. Animals receive the aerosol administration of agonist for a maximum time of 780 seconds.

Differences between drug-treated groups and corresponding vehicle-treated control groups are compared using Student's t-test for unpaired observations. Results are reported as % protection values, where

% protection =

[(drug time - mean control time)/(maximal aerosol time - mean control time)] X 100

Clinical Studies

Clinical studies to demonstrate the efficacy of a Compound of the invention may be carried out using standard methods. For example, the ability of a Compound to prevent or treat the symptoms of asthma or asthma-like conditions may be demonstrated using a challenge of inhaled cold air or allergen and evaluation by standard pulmonary measurements such as, for example, $FEV_1$ (forced expiratory volume in one second) and FVC (forced vital capacity), analyzed by standard methods of statistical analysis.

It will be appreciated that the implications of a Compound's activity in the above desribed Tests is not limited to asthma, but rather, that the Tests provide evidence of general antagonism of both SP and NKA

SP and NKA have been implicated in the pathology of numerous diseases including: rheumatoid arthritis, Alzheimer's disease, oedema, allergic rhinitis, inflamation pain, gastrointestinal-hypermotility, anxiety, emesis, Huntington's Disease, Psycoses, hypertension, migraine, bladder hypermotility and uticaria. Accordingly, one feature of the invention is the use of a compound of formula I or a pharmaceutically acceptable salt thereof in the treatment of a disease in a human or other mammal in need thereof in which SP or NKA is implicated and antagonism of its action is desired.

Asthma is characterized by both chronic inflammation and hyperresponsiveness of the airways. The NK1 receptor is known to mediate inflammation and mucus hypersecretion in airways; and the NK2 receptor is involved in the control of the tone of bronchial smooth muscle. Thus, agents capable of antagonizing the actions of SP and NKA, at the NK1 and NK2 receptors, respectively, are capable of reducing both the chronic inflammation and the airway hyperresponsiveness which are symptomatic of asthma. It has been suggested that an antagonist having mixed affinity for NK1 and NK2 could be therapeutically superior to a receptor selective antagonist. C.M. Maggi "Tachykinin Receptors and Airway Pathophysiology" EUR. Respir. J., 1993, 6, 735-742 at 739. Also, it has been suggested that a synergistic

effect against bronchoconstriction may result from the simultaneous application of an NK1 antagonist and an NK2 antagonist. D.M. Foulon, et al. "NK1 and NK2 Receptors Mediated Tachykinin and Resiniferatoxin-induced Bronchospasm in Guinea Pigs" American Review of Respiratory Disease, 1993, 148, 915-921. Accordingly, another feature of the invention is the use of a compound of formula I or a pharmaceutically acceptable salt thereof in the treatment of asthma in a human or other mammal in need thereof. Because of the range of effects attributable to the actions of SP and NKA, compounds which are capable of blocking their actions may also be useful as tools for further evaluating the biological actions of other neurotransmitters in the Tachykinin family. As a result, another feature of the invention is provided by the use of a compound of formula I or a salt thereof as a pharmacological standard for the development and standardization of new disease models or assays for use in developing new therapeutic agents for treating diseases in which SP or NKA are implicated or for assays for their diagnosis.

When used in the treatment of a disease, a compound of the invention is generally administered as an appropriate pharmaceutical composition which comprises a compound of formula I or a pharmaceutically acceptable salt thereof as defined hereinbefore and a pharmaceutically acceptable diluent or carrier, the composition being adapted for the particular route of administration chosen. Such a composition is provided as a further feature of the invention. It may be obtained employing conventional procedures and excipients and binders, and it may be one of a variety of dosage forms. Such forms include, for example, tablets, capsules, solutions or suspensions for oral administration; suppositories for rectal administration; sterile solutions or suspensions for administration by intravenous or intramuscular infusion or injection; aerosols or nebulizer solutions or suspensions for administration by inhalation; or powders together with pharmaceutically acceptable solid diluents such as lactose for administration by insufflation.

For oral administration a tablet or capsule containing up to 250 mg (and typically 5 to 100 mg) of a compound of formula I may conveniently be used. For administration by inhalation, a compound of formula I will be administered to humans in a daily dose range of, for example, 5 to 100 mg, in a single dose or divided into two to four daily doses. Similarly, for intravenous or intramuscular injection or infusion a sterile solution or suspension containing up to 10% w/w (and typically 0.05 to 5% w/w) of a compound of formula I may conveniently be used.

The dose of a compound of formula I to be administered will necessarily be varied according to principles well known in the art taking account of the route of administration and the severity of the condition and the size and age of the patient under treatment. However, in general, the compound of formula I will be administered to a warm-blooded animal (such as man) so that a dose in the range of, for example, 0.01 to 25 mg/kg (and usually 0.1 to 5 mg/kg) is received. It will be understood that generally equivalent amounts of a pharmaceutically acceptable salt of a compound of formula I may be used.

The invention will now be illustrated by the following non-limiting examples in which, unless stated otherwise:

(i) temperatures are given in degrees Celsius (°C); operations were carried out at room or ambient temperature, that is, at a temperature in the range of 18-25 °C;

(ii) organic solutions were dried over anhydrous magnesium sulfate; evaporation of solvent was carried out using a rotary evaporator under reduced pressure (600-4000 pascals; 4.5-30 mm Hg) with a bath temperature of up to 60 °C;

(iii) chromatography means flash chromatography on silica gel; thin layer chromatography (TLC) was carried out on silica gel plates;

(iv) in general, the course of reactions was followed by TLC and reaction times are given for illustration only;

(v) melting points are uncorrected and (dec) indicates decomposition; the melting points given are those obtained for the materials prepared as described; polymorphism may result in isolation of materials with different melting points in some preparations;

(vi) final products had satisfactory proton nuclear magnetic resonance (NMR) spectra;

(vii) yields are given for illustration only and are not necessarily those which may be obtained by diligent process development; preparations were repeated if more material was required;

(viii) when given, NMR data is in the form of delta values for major diagnostic protons, given in parts per million (ppm) relative to tetramethylsilane (TMS) as an internal standard, determined at 300 MHz using deuterated chloroform ($CDCl_3$) as solvent; conventional abbreviations for signal shape are used; for AB spectra the directly observed shifts are reported; coupling constants (J) are given in Hz; Ar designates an aromatic proton when such an assignment is made;

(ix) chemical symbols have their usual meanings; SI units and symbols are used;

(x) reduced pressures are given as absolute pressures in pascals (Pa); elevated pressures are given as gauge pressures in bars;

(xi) solvent ratios are given in volume:volume (v/v) terms; and

(xii) Mass spectra (MS) were run using an automated system with atmospheric pressure chemical ionization (ApCI). Methanol mobile phase enters the probe were it is pneumatically converted into an aerosol and rapidly heated into the gas phase at the probe tip. Hot gas from the probe enters the heated volume of the source which contains the corona discharge pin typically maintained at 3 kV. Methanol molecules rapidly react with ions from the corona discharge to produce stable reagent ions. Analyte molecules introduced into the mobile phase react with the reagent ions at atmospheric pressure and typically become protonated (for positive ions) or deprotonated (for negative ions). Where indicated, the following alternative methods of ionization were used; a) desorption chemical ionization (CI) using methane reagent gas and a direct exposure probe; b) electron impact (EI) or c) fast atom bombardment (FAB). Generally, only spectra where parent masses are observed are reported.

EXAMPLES

Example 1. N-Benzyl-5-(4-hydroxy-4-phenylpiperidino)-3-(3,4-dichlorophenyl)pentanamide.

To a solution of benzylamine (191 mg) in toluene (2 mL) cooled to 0 °C was added trimethylaluminum (0.66 mL of a 2.0 M solution in toluene). The mixture was allowed to warm to room temperature over two hours. To this stirred solution was added ethyl 5-(4-hydroxy-4-phenyl-piperidino)-3-(3,4-dichlorophenyl)pentanoate (300 mg) in toluene. The solution was warmed to 60 °C for two hours and then stirred at room temperature overnight. The solution was diluted with dichloromethane and added to a mixture of brine and dichloromethane. The organic layer was separated and washed with brine. The aqueous layers were extracted with dichloromethane. The combined organic layers were dried, filtered and evaporated to afford an oil. Chromatography, with chloroform:methanol:ammonium hydroxide (97:3:0.25) as the eluent gave an oil which crystallized upon exposure to diethyl ether:hexane to give the title compound (57 mg); mp 64-67 °C; MS (CI): m/z=513(M+1); NMR ($CDCl_3$, $CF_3COOD$): 1.27 (1,m), 2.22 (3,m), 2.39 (2,m), 2.77 (3,m), 3.15 (2,m), 3.32 (2,m), 3.56 (2,m), 4.21 (1,d), 4.45 (1,d), 6.96 (3,m), 7.34 (10,m) . Analysis for $C_{29}H_{32}N_2O_2Cl_2$: Calculated: C, 68.09; H, 6.30; N, 5.47; Found: C, 68.01; H, 6.53; N, 5.24.

The intermediate ethyl 5-(4-hydroxy-4-phenylpiperidino)-3-(3,4-dichlorophenyl)-pentanoate was prepared as follows.

a. Ethyl 4,4-bis(tert-butoxycarbonyl)-3-(3,4-dichlorophenyl)-butanonate. To a stirred solution of bis(tert-butyl) malonate (13.7 g) in tetrahydrofuran (100 mL) was added potassium tert-butoxide (53.1 mL of a 1M solution in tetrahydrofuran). This mixture was stirred for 20 minutes and to it was added ethyl (E)-3-(3,4-dichloro-phenyl)-2-propenoate (12.96 g as a solution in tetrahydrofuran (30 mL)). The solution was stirred at room temperature for 1.5 hours and then diluted sequentially with diethyl ether (30 mL) and brine (30 mL). The organic layer was separated, dried, filtered, and evaporated to afford the tri-ester (22 g) as an oil; NMR: 7.53 (m,2), 7.28 (d,1, J=8.34), 3.91 (q,2, J=5.17), 3.8 (m,1), 3.55 (m,1), 2.72 (m,2), 1.4 (broad s,9), 1.13 (s,9), 1.01 (t,3, J=6.9).

b. Ethyl 4-carboxy-3-(3,4-dichlorophenyl)butanoate. Neat ethyl 4,4-bis(tert-butoxycarbonyl)-3-(3,4-dichlorophenyl)butanonate (22 g) was slowly heated to 180 °C for 4 hours. The material was allowed to cool to room temperature to yield the acid (14.4 g); NMR: 7.56 (m,2), 7.29 (dd,1, J=1.84, 8.35), 3.9 (q,2, J=7.07), 3.39 (m,1), 2.6 (m, 4), 1.08 (t,3, J=7.03).

c. Ethyl 3-(3,4-dichlorophenyl)-5-hydroxypentanoate. Borane-methyl sulfide (1.76 mL of a 2.0 M solution in tetrahydrofuran) was added to a solution of ethyl 4-carboxy-3-(3,4-dichlorophenyl)-butanoate (5.34 g) in tetrahydrofuran (100 mL) over 20 minutes. The mixture was warmed to 50 °C for 2 hours. The solution was cooled to room temperature and diluted sequentially with water (100 ml) and ethyl acetate (350 ml). The organic layer was separated and the aqueous layer was extracted with ethyl acetate (100 mL). The combined organic layers were dried, filtered and evaporated to afford an oil. Chromatography with hexane:acetone (70:30) as eluent gave the alcohol as an oil (4.70 g); NMR: 7.3 (m,2), 7.07 (dd,1, J=2.14, 8.25), 4.0 (q,2, J=7.18), 3.5 (m,2), 3.0 (m,1), 2.6 (m,2), 1.9 (m,2), 1.16 (t,3, J=8.74).

d. Ethyl 3-(3,4-dichlorophenyl)-5-oxopentanoate. Pyridinium chlorochromate (3.5 g) was added to a solution of

ethyl 3-(3,4-dichlorophenyl)-5-hydroxypentanoate (4.65 g) in dichloromethane (75 mL), at 0 °C. The stirred solution was brought to reflux for 1.5 hours. Additional pyridium chlorochromate (0.3 g) was added to the mixture, and it was brought back to reflux for an addition 0.75 hours. The mixture was cooled and filtered through FLORISIL [FLORISIL was purchased from the Aldrich Chemical Company of Milwaukee, Wisconsin, catalogue number 34,399-4]. The resulting filtrates were evaporated to give the aldehyde (3.75 g) as an oil that was used without further purification; NMR: 9.6 (s,1), 7.3 (m,2), 7.0 (m, 1), 4.0 (m,2), 3.7 (m,1), 2.6 (m,4), 1.18 (m,3).

e. Ethyl 5-(4-hydroxy-4-phenylpiperidino)-3-(3,4-dichloro-phenyl)pentanoate. 4-Hydroxy-4-phenylpiperidine (2.74 g) was added to a solution of ethyl 3-(3,4-dichlorophenyl)-5-oxopentanoate (3.74 g) in methanol (80 mL) and the mixture was cooled to 0 °C. Glacial acetic acid was added until the pH was 5.5. Sodium cyanoborohydride (total 0.97 g) was added at one hour intervals in three equal portions. The mixture was allowed to warm to room temperature overnight. The mixture was diluted sequentially with aqueous sodium hydroxide (1N, 100 mL) and dichloromethane (200 mL). The organic layer was separated and extracted with brine. The aqueous layers were extracted with dichloromethane (3 x 200 mL). The combined organic layers were dried, filtered and evaporated to give an oil. Chromatography with chloroform:methanol:ammonium hydroxide (97:3:0.25) as the eluent gave the ester (1.6 g) as an oil; NMR: 7.3 (m,8), 4.0 (q,2, J=7.22), 3.1 (m,1), 2.0 (m,14), 1.17 (m,3).

Ethyl 5-(4-hydroxy-4-phenylpiperidino)-3-(3,4-dichloro-phenyl)pentanoate, described in sub-part e. above, is also a compound of the invention.

Example 2. 1-[5-(4-hydroxy-4-phenylpiperidino)-3-(3,4-dichloro-phenyl)valeryl]-4-phenylpiperidine hydrochloride salt.

To a solution of 1-[5-(4-hydroxy-4-phenylpiperidino)-3-(3,4-dichlorophenyl)valeryl]-4-phenylpiperidine (0.18 g) in diethyl ether (20 mL) was added a solution of hydrochloric acid in diethyl ether (0.15 mL, 2N HCl). The solvent was evaporated to afford a solid. The solid was triterated two times with diethyl ether (20 mL) and dried overnight under reduced pressure to give the title salt as an off white solid (141.52 mg); mp 135-142 °C; MS (CI): m/z=567(M+1); NMR: 1.47 (m,6), 2.31 (m,4), 3.04 (m,12), 4.03 (m,1), 4.61 (broad d,1), 7.32 (m, 13). Analysis for $C_{33}H_{38}Cl_2N_2O_2$•HCl•0.5 $H_2O$: Calculated: C, 65.94; H, 6.37; N, 4.66; Found: C, 64.98; H, 6.46; N, 4.34.

The intermediate 1-[5-(4-hydroxy-4-phenylpiperidino)-3-(3,4-dichlorophenyl)valeryl]-4-phenylpiperidine, which is also a compound of the invention, was prepared using a procedure similar to that described in Example 1, except replacing the benzyl amine used therein with 4-phenylpiperidine.

Example 3-8

Using a procedure similar similar to that described in Example 2, except replacing the 1-[5-(4-hydroxy-4-phenylpiperidino)-3-(3,4-dichlorophenyl)valeryl]-4-phenylpiperidine used therein with the requsite compound, the hydrochloride salts of the following compounds of formula I wherein $Q^1$ is 4-hydroxy-4-phenylpiperidine, $Q^2$ is 3,4-dichlorophenyl, $Q^3$ is hydrogen and $Q^4$ has the indicated values were prepared.

Example 3. $Q^4$=4-Methoxybenzylamino; mp 104-110 °C; MS (CI): m/z=543(M+1); NMR ($CD_3OD$): 1.97 (m, 2), 2.23 (m,4), 2.61 (m,2), 2.84 (m,1), 3.33 (m,6), 3.75 (s,3), 3.99 (m,1), 4.33 (m,1), 6.81 (m,4), 7.35 (m,8). Analysis for $C_{30}H_{34}N_2Cl_2O_3$•HCl: Calculated: C, 62.34; H, 6.10; N, 4.84; Found: C, 62.16; H, 6.17; N, 4.90.

Example 4. $Q^4$=Cyclohexylamino; mp 112-117 °C; MS (CI): m/z=505(M+1); NMR ($CD_3OD$): 1.19 (m,5), 1.68 (m,5), 1.97 (m,2), 2.45 (m,6), 2.91 (m,1), 3.30 (m,7), 7.4 (m,8). Analysis for $C_{20}H_{36}Cl_2N_2O_2$•HCl•0.4 $H_2O$: Calculated: C, 62.28; H, 6.90; N, 5.18; Found: C, 61.50; H, 6.76; N, 4.82.

Example 5. $Q^4$=(4-Methylbenzyl)amino; mp 115-120 °C; MS (CI): m/z=527(M+1); NMR ($CD_3OD$): 1.97 (m, 2), 2.29 (m,7), 2.63 (m,2), 2.88 (m,1), 3.29 (m,6), 4.03 (m,1), 4.32 (m,1), 6.83 (d,2), 7.03 (d,2), 7.34 (m,8). Analysis for $C_{30}H_{34}N_2Cl_2O_2$•HCl: Calculated: C, 64.11; H, 6.27; N, 4.98; Found: C, 63.98; H, 6.36; N, 5.05.

Example 6. $Q^4$=(Benzyl)(methyl)amino; mp 74-81 °C; MS (CI): m/z=527(M+1); NMR ($CD_3OD$): 2.10 (m,6), 2.87 (m,6), 3.39 (m,6), 4.31-4.66 (m,2), 7.3 (m,13). Analysis for $C_{30}H_{34}Cl_2N_2O_2$•HCl•0.5 $H_2O$: Calculated: C, 64.11; H, 6.27; N, 4.98; Found: C, 63.02; H, 6.22; N, 4.86.

Example 7. $Q^4$=(Methyl)(phenyl)amino: mp 96-102 °C; MS (CI): m/z=513(M+1); NMR ($CD_3OD$): 2.0 (broad m,6), 2.48 (broad d,2), 3.14 (m,7), 3.38 (m,3), 7.03 (m,3), 7.37 (m,11). Analysis for $C_{29}H_{32}Cl_2N_2O_2$•HCl•0.5 $H_2O$: Calculated: C, 63.56; H, 6.07; N, 5.11; Found: C, 62.68; H, 6.02; N, 5.05.

Example 8. $Q^4$=Anilino; mp 113-120 °C; MS (CI): m/z=499(M+1); NMR: 1.98 (m,24), 2.29 (m,4), 2.77 (m,3), 3.32 (m,6), 7.37 (m,13). Analysis for $C_{28}H_{30}Cl_2N_2O_2$•HCl•0.4 $H_2O$: Calculated: C, 62.98; H, 5.85; N, 5.24; Found: C, 62.17; H, 5.82; N, 5.49.

The intermediate amides used in Examples 3-8 were prepared using a sequence similar to that described in Example 1 and the sub-parts thereof, except replacing the benzylamine used therein with the requsite amine.

Example 9. Benzyl 5-(4-acetamido-4-phenylpiperidino)-3-(3,4-dichlorophenyl)pentanoate.

Benzyl 3-(3,4-dichlorophenyl)-5-oxopentanoate (0.850 g) was added to a solution of 4-acetamido-4-phenylpiperidine (0.58 g) in methanol (20 mL) and stirred for 5 minutes. Sodium cyanoborohydride (0.157 g) was added and the mixture adjusted to pH 5 by the addition of 5 drops of glacial acetic acid. The mixture was stirred at room temperature for 1.5 hours, and was evaporated. The resulting material was diluted with ethyl acetate, washed (saturated sodium bicarbonate, brine), dried, and evaporated to afford an oil. Chromatography with dichloromethane:methanol:ammonium hydroxide (95:5:0.1) as the eluent gave the title compound as a foamy white solid (0.52 g); mp 62-64 °C; MS (CI): m/z=555(M+1); NMR: 1.70 (m,1), 1.86 (m,1), 2.00 (s,3), 2.14 (m,6), 2.34 (m,2), 2.66 (m,4), 3,18 (m,1), 5.02 (s,2), 5.47 (s,1), 7.01 (m,1), 7.01-7.40 (m,12). Analysis for $C_{31}H_{34}Cl_2N_2O_3$•0.5 $H_2O$: Calculated: C, 66.19; H, 6.27; N, 4.98; Found: C, 66.35; H, 6.09; N, 4.78.

The intermediate benzyl 3-(3,4-dichlorophenyl)-5-oxopentanoate was prepared as follows.

a. Benzyl 3-(3,4-dichlorophenyl)-2-propenoate. 3-(3,4-dichlorophenyl)-2-propenoic acid (20.0 g) was suspended in thionyl chloride (60 mL) and heated at reflux for 2 hours. The mixture was evaporated and the residue dissolved in dichloromethane (100 mL). To this solution was added benzyl alcohol (10.9 g) at 0 °C. The mixture was stirred at room temperature for 2 hours, diluted with an additional 100 mL of dichloromethane and washed with 1M HCl (100 mL), 1M NaOH (150 mL) and brine (100 mL). The organic phase was dried and evaporated. Recrystallization from diethyl ether:hexanes gave the ester (20.9 g) as a white solid.

b. Benzyl 4,4-bis(tert-butoxycarbonyl)-3-(3,4-dichlorophenyl)-butanonate. bis(Tert-butyl) malonate (16.3 g) in tetrahydrofuran (100 mL) was added to a solution of potassium tert-butoxide (79 mL, 1M in tert-butanol) and tetrahydrofuran (100 mL) dropwise at 10 °C. The mixture was allowed to warm gradually to room temperature and stirred an additional 40 minutes. The reaction was cooled to 5 °C and a solution of benzyl 3-(3,4-dichlorophenyl)-2-propenoate (21.0 g) in tetrahydrofuran (75 mL) was added dropwise over 15 minutes. The mixture was allowed to warm and stir at room temperature for 3 hours. A solution of saturated aqueous potassium phosphate monobasic (60 mL) was added to the reaction. The mixture was stirred 10 minutes, and was transfered to a separatory funnel containing ethyl ether (500 mL) and saturated aqueous potassium phosphate monobasic (100 mL). The ether phase was separated and the aqueous phase extracted with ether. The ether extracts were combined, washed (saturated sodium bicarbonate, brine), dried, and evaporated to give the tri-ester as an off-white solid (36.9 g); NMR: 7.31-7.03 (m,8), 4.97 (s,2), 3.75 (m,1), 3.45 (d,1, J=10), 3.00-2.90 (m,1), 2.73-2.49 (m,1), 1.45 (s,9), 1.23 (s,9); TLC: Rf = 0.72 (1:1 diethyl ether:hexane).

c. Benzyl 4-carboxy-3-(3,4-dichlorophenyl)butanoate. Benzyl 4,4-bis(tert-butoxycarbonyl)-3-(3,4-dichlorophenyl) butanonate (36.05 g) was treated with trifluoroacetic acid (150 mL) at 0 °C. The resulting suspension was warmed to room temperature and treated with dichloromethane (25 mL) to aid dissolution. After 1 hour the mixture was evaporated to afford a reddish oil, which was heated at 120 °C for 3 hours and then heated under vacuum (120 °C, 67 Pa) for 3 hours. The oil solidified upon cooling to afford the acid as a tan solid (22.8 g); NMR: 7.40-7.00 (m, 8), 5.02 (s,2), 3.60 (m,1), 2.81-2.59 (m,4); MS (CI): m/z=367 (M+1); TLC: Rf = 0.21 (10:90:0.1 methanol:dichloromethane:ammonium hydroxide).

d. Benzyl 3-(3,4-dichlorophenyl)-5-hydroxypentanoate. Benzyl 4-carboxy-3-(3,4-dichlorophenyl)butanoate (7.34 g) was dissolved in tetrahydrofuran (100 mL). Borane-tetrahydrofuran complex (21 mL x 1M in tetrahydrofuran) was added dropwise and the solution was stirred for 1 hour at 0 °C. The mixture was heated at reflux for 1.5 hours. Additional borane-tetrahydrofuran complex (5 mL x 1M in tetrahydrofuran) was added at room temperature, and the reaction was again allowed to reflux for 1 hour. Saturated sodium bicarbonate (10 mL) was added to the reaction at 0 °C. The mixture was diluted with diethyl ether, mL), washed (saturated sodium bicarbonate, brine), dried, and evaporated to afford the alcohol as a yellow oil (7.07 g); NMR: 7.38-7.00 (m,8), 5.02 (s,1), 3.58-3.25 (m,3), 2.75-2.58 (m,2), 1.98-1.74 (m,3); TLC: Rf = 0.15 (1:1 diethyl ether:hexane).

e. Benzyl 3-(3,4-dichlorophenyl)-5-oxopentanoate. Benzyl 3-(3,4-dichlorophenyl)-5-hydroxypentanoate (1.60 g) was added to a dichloromethane (50 mL) solution containing N-methylmorpholine-N-oxide (0.80 g) and 4 Angstrom molecular sieves. The mixture was stirred for 15 minutes and tetrapropylammonium perruthenate (0.09 g) was added. After 1.5 hours, the mixture was diluted with dichloromethane (100 mL) washed (10% aqueous sodium sulfite, hydrochloric acid (1M x 50 mL), saturated sodium bicarbonate, brine), dried, and evaporated. Chromatographed through a short FLORISIL plug [FLORISIL was purchased from the Aldrich Chemical Company of Milwaukee, Wisconsin, catalogue number 34,399-4] eluting with diethyl ether gave the aldehyde as a colorless oil (0.85 g); NMR: 9.67 (s,1), 7.45-7.18 (m,7), 7.03 (m,1), 5.03 (s,2), 3.71 (m,1), 2.80-2.58 (m,4); TLC: Rf = 0.26 (1:

1 ethyl ether:hexane).

Example 10. 5-(4-Acetamido-4-phenylpiperidino)-3-(3,4-dichloro-phenyl)-N-(2-methoxybenzyl)-N-methylpentanamide.

5-(4-Acetamido-4-phenylpiperidino)-3-(3,4-dichlorophenyl)pentanoic acid (0.60 g) and N-(2-methoxybenzyl)-N-(methyl)amine hydrochloride (0.225 g) were combined in dry N,N-dimethylformamide (13 mL) under nitrogen. Triethylamine (0.121 g) was added followed by 1-(3-dimethylaminopropyl)-3-ethylcarbodiimide methiodide (0.595 g). The mixture was stirred overnight. The mixture was diluted with ethyl acetate and partitioned with saturated sodium bicarbonate. The aqueous phase was extracted with ethyl acetate and the organic extracts were combined, washed (brine), dried, and evaporated to afford an oil. Chromatography with dichloromethane:methanol:ammonium hydroxide (95:5:0.1) as the eluent gave the title compound as a white solid (0.248 g); mp 96-98 °C; MS (CI): m/z=598(M+1); NMR: 1.90-2.34 (m,10), 2.00 (s,3), 2.61-2.73 (m,4), 2.88 (s,3), 3.31 (m,1), 3.78 and 3.82 (s,3, two signals due to rotational isomers), 4,42-4.66 (m,2), 5.46 (broad,1), 6.80-6.93 (m,3), 7.02-7.39 (m,9). Analysis for $C_{33}H_{39}N_3Cl_2O_3$•0.20 $H_2O$: Calculated: C, 66.04; H, 6.62; N, 7.00; Found: C, 66.14; H, 6.57; N, 6.98.

The intermediate 5-(4-acetamido-4-phenylpiperidino)-3-(3,4-dichlorophenyl)pentanoic acid was prepared as follows.

a. 5-(4-Acetamido-4-phenylpiperidino)-3-(3,4-dichlorophenyl)pentanoic acid. Benzyl 5-(4-acetamido-4-phenylpiperidino)-3-(3,4-dichlorophenyl)pentanoate (0.100 g) was dissolved in ethanol (35 mL) containing 10% Pd on carbon (0.050 g). The mixture was hydrogenated (2.4 bar, 1 hour). The resulting mixture was filtered through diatomaceous earth with ethanol. Evaporation of the resulting solution gave the acid as a waxy yellow solid (0.070 g), which had an NMR similar to the material prepared in sub-part b. below.

The intermediate 5-(4-acetamido-4-phenylpiperidino)-3-(3,4-dichlorophenyl)pentanoic acid can alternativly be prepared as follows.

b. 5-(4-Acetamido-4-phenylpiperidino)-3-(3,4-dichlorophenyl)pentanoic acid. To a solution of ethyl 5-(4-acetamido-4-phenyl-piperidino)-3-(3,4-dichlorophenyl)pentanoate (1.00 g) (prepared as described in Example 15) in tetrahydrofuran (10 mL), and methanol (10 mL) was added a solution of lithium hydroxide monohydrate (0.22 g) in water (10 mL). The mixture was stirred for 16 hours, was acidified by addition of 1M HC1, and evaporated to afford the acid as a white powder (1.3 g); NMR: 8.42 (s,1), 7.62-7.55 (m,2), 7.35-7.31 (m,5), 7.22 (m,1), 3.35 (m,1), 3.14-2.95 (m,4), 2.79-2.30 (m,6), 2.16 (m,2), 2.09 (s,3), 1.91 (m,2); MS (CI): m/z=463(M+1); TLC: Rf=0.51 (10:1:0.1 tetrahydrofuran: water:acetic acid).

The intermediate 5-(4-acetamido-4-phenylpiperidino)-3-(3,4-dichlorophenyl)pentanoic acid is also a compound of the invention.

Examples 11-14

Using a procedure similar to that described in Example 10, except replacing the N-methyl-N-(2-methoxybenzyl)amine with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined were prepared.

Example 11. $Q^4$=Benzylamino; mp 173-174 °C; MS (CI): m/z=554(M+1); NMR (perdeuteriodimethylsulfoxide): 1.91 (s,3), 2.10-2.27 (m,4), 2.55 (m,3), 2.77 (m,2), 2.98-3.18 (m,5), 3.40 (m,3), 3.99-4.06 (m,1), 4.29-4.36 (m,1), 7.18-7.44 (m,9), 7.56-7.61 (m,2), 8.12 (broad,1), 8.36 (broad t,1), 10.26 (broad,1). Analysis for $C_{31}H_{35}Cl_2N_3O_2$•1.00 $H_2O$•1.00 HCl: Calculated: C, 61.33; H, 6.31; N, 6.92; Found: C, 61.12; H, 6.43; N, 7.19.

Example 12. $Q^4$=(Methyl)[3,5-bis(trifluoromethyl)benzyl]amino; mp 84-86 °C; MS (CI): m/z=704(M+1); NMR: 1.72 (m,1), 2.00 (s,3), 2.00-2.36 (m,9), 2.70 (m,4), 2.93 (s,3), 3.33 (m,1), 4.61 (m,2), 5.46 (s,1), 7.10 (m,1), 7.19-7.39 (m,7), 7.56 (m,2), 7.81 (m,1). Analysis for $C_{34}H_{35}Cl_2F_6N_3O_2$: Calculated: C, 58.13; H, 5.02; N, 5.98; Found: C, 58.07; H, 5.16; N, 5.80.

Example 13. $Q^4$=(Methyl)(benzyl)amino; mp 88-90 °C; MS (CI): m/z=568(M+1); NMR: 1.76 (m), 1.95-2.35 (m,9), 2.00 (s,3), 2.62-2.74 (m,4), 2.86 (d,3), 3.34 (m,1), 4.35-4.66 (m,2), 5.45 (broad,1), 6.95-7.40 (m,13). Analysis for $C_{32}H_{37}Cl_2N_3O_2$•1.00 $H_2O$: Calculated: C, 65.75; H, 6.72; N, 7.19; Found: C, 65.59; H, 6.34; N, 7.39.

Example 14. $Q^4$=(2-Methoxybenzyl)amino; mp 102-104 °C; MS (CI): m/z=582(M+1); NMR: 1.69 (m,2), 1.90-2.35 (m,9), 2.00 (s,3), 2.48-2.75 (m,3), 3.18 (m,1), 3.78 (s,3), 4.30 (m,2), 5.48 (broad,1), 5.77 (broad,1), 6.80-7.05 (m,4), 7.19-7.39 (m,8). Analysis for $C_{32}H_{37}Cl_2N_3O_3$•0.30 $H_2O$: Calculated: C, 65.37; H, 6.44; N, 7.15; Found: C, 65.34; H, 6.36; N, 7.21. Example 15. Ethyl 5-(4-acetamido-4-phenylpiperidino)-3-(3,4-dichlorophenyl)pentanoate.

Using a sequence similar to that described in Example 9 and the sub-parts thereof, except replacing benzylalcohol with ethanol in sub-part a., the title compound was prepared as a white solid; mp 91-100 °C; NMR ($CDCl_3$, $CF_3COOD$): 7.64-7.59 (m,2), 7.4-7.3 (m,5), 7.30-7.20 (m,1), 4.0 (q,2), 3.46 (t,2), 3.23-3.03 (m,4), 2.93-2.67 (m,4), 2.17-2.0 (m,5), 1.9 (s,3), 1.1 (t,3); MS (CI): m/z=493(M+1); TLC: $R_f$=0.27 (95:5, dichloromethane:methanol). Analysis for $C_{26}H_{32}Cl_2N_2O_3$•0.50 $H_2O$: Calculated: C, 62.39; H, 6.65; N, 5.60; Found: C, 62.74; H, 6.53; N, 5.53.

Example 16. 4-Acetamido-1-[3-(3,4-dichlorophenyl)-4-(1,2,3,4-tetrahydroisoquinol-2-ylcarbonyl)butyl]-4-phenylpiperidine.

5-(4-Acetamido-4-phenylpiperidino)-3-(3,4-dichlorophenyl)pentanoic acid (0.276 g) in dimethylformamide (5 mL) was added to 1,2,3,4-tetrahydroisoquinoline (0.075 g), in a 16x100 mm tube. The reaction tube was vortexed to give a solution. 1-(3-dimethylaminopropyl)-3-ethylcarbodiimide hydrochloride (0.106 g) was added and the mixture was stirred overnight at room temperature. The solvent was removed by centrifugal evaporation and the residue was partitioned between ethyl acetate (3 mL) and saturated aqueous sodium bicarbonate solution (4 mL). The organic phase was separated and the aqueous phase was extracted with ethyl acetate (2x3 mL). The combined organic extracts were evaporated to give material which was purified by chromatography using a Bond elut silica column, to give the title compound; MS: m/z=578(M+1). Analysis for $C_{33}H_{37}Cl_2N_3O_2$•1.0 $H_2O$: Calculated: C, 66.44; H, 6.59; N, 7.04; Found: C, 66.43; H, 6.39; N, 6.80. The procedure may conveniently be carried out with the assistance of a robot.

Examples 17-54

Using a procedure similar to that described in Example 16, except replacing the 1,2,3,4-tetrahydroisoquinoline with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined, were prepared. Reaction products were analysed by high performance liquid chromatography on a HYPERSIL ODS column (4.6 x 250 mm), using a flow rate of 1.5 mL/minute, and UV detection (280 nm), with a column temperature of 40 °C. The eluents were prepared as follows:

Solvent 1 = 0.1% trifluoroacetic acid in water
Solvent 2 = 0.1% trifluoroacetic acid in acetonitrile
Solvent 3 = 1 mM triethylamine in water
Solvent 4 = 1 mM triethylamine in acetonitrile

Solvent A, linear gradient : time 0 minutes, 5:95 (Solvent 4:Solvent 3); time 3.0 minutes, 5:95 (Solvent 4:Solvent 3); time 17.0 minutes, 95:5 (Solvent 4; Solvent 3); time 18 minutes, 5:95 (Solvent 4:Solvent 3).

Solvent B, linear gradient: time 0 minutes, 75:25 (Solvent 2:Solvent 1); time 17.0 minutes, 75:25 (Solvent 2; Solvent 1); time 18 minutes, 95:5 (Solvent 2:Solvent 1); time 20 minutes, 75:25 (Solvent 2:Solvent 1).

Example 17. $Q^4$=4-Carbamoyl-4-phenylpiperidino; MS: m/z=649(M+1); HPLC: Solvent A, rt=11.07.
Example 18. $Q^4$=3-Phenyl-1-propylamino; MS: m/z=580(M+1); HPLC: Solvent A, rt=11.70.
Example 19. $Q^4$=3-Chlorobenzylamino; MS: m/z=586(M+1); HPLC: Solvent B, rt=15.27.
Example 20. $Q^4$=2-Thienylmethylamino; MS: m/z=558(M+1); HPLC: Solvent B, rt=6.86.
Example 21. $Q^4$=4-(Aminosulfonyl)phenethylamino; MS: m/z=645(M+1); HPLC: Solvent B, rt=4.54.
Example 22. $Q^4$=4-Phenyl-1,2,3,6-tetrahydropyrid-1-yl; MS: m/z=604(M+1); HPLC: Solvent B, rt=21.66.
Example 23. $Q^4$=4-(Dimethylamino)benzylamino; MS: m/z=595(M+1); HPLC: Solvent B, rt=6.06.
Example 24. $Q^4$=3,5-Dimethoxyanilino; MS: m/z=598(M+1); HPLC: Solvent B, rt=13.98.
Example 25. $Q^4$=3-(Imidazol-1-yl)propylamino; MS: m/z=570(M+1); HPLC: Solvent B, rt=6.13.
Example 26. $Q^4$=α-(Hydroximino)phenethylamino; MS: m/z=595(M+1); HPLC: Solvent B, rt=8.96.
Example 27. $Q^4$=2-(Imidazol-4-yl)ethylamino; MS: m/z=556(M+1); HPLC: Solvent B, rt=6.18.
Example 28. $Q^4$=3-Hydroxy-3-phenylpyrrolidin-1-yl; MS: m/z=608(M+1); HPLC: Solvent B, rt=7.56.
Example 29. $Q^4$=N-(Fur-2-ylmethyl)-N-methylamino; MS: m/z=556(M+1); HPLC: Solvent B, rt=12.85.
Example 30. $Q^4$=Fur-2-ylmethylamino; MS: m/z=542(M+1); HPLC: Solvent B, rt=6.13.
Example 31. $Q^4$=2-(Indol-3-yl)ethylamino; MS: m/z=605(M+1); HPLC: Solvent B, rt=12.55.
Example 32. $Q^4$=2-(5-Fluoroindol-3-yl)ethylamino; MS: m/z=623(M+1); HPLC: Solvent B, rt=12.83.
Example 33. $Q^4$=3,4-(methylenedioxy)benzylamino; MS: m/z=596(M+1); HPLC: Solvent B, rt=10.71.
Example 34. $Q^4$=4-Hydroxy-4-phenylpiperidino; MS: m/z=622(M+1); HPLC: Solvent B, rt=10.22.
Example 35. $Q^4$=Indan-1-ylamino; MS: m/z=578(M+1); HPLC: Solvent B, rt=15.60.
Example 36. $Q^4$=4-Phenylpiperidino; MS: m/z=606(M+1); HPLC: Solvent B, rt=22.10.
Example 37. $Q^4$=4-Acetamidomethyl-4-phenylpiperidino; MS: m/z=677(M+1);

Example 38. $Q^4$=N-Methyl-N-(2-pyridylmethyl)amino; MS: m/z=567(M+1); HPLC: Solvent B, rt=7.05.

Example 39. $Q^4$=N-Methyl-N-(6-methylpyrid-2-ylmethyl)amino; MS: m/z=581(M+1); HPLC: Solvent B, rt=7.28.

Example 40. $Q^4$=2-(Pyrid-2-yl)ethylamino; MS: m/z=567(M+1); HPLC: Solvent B, rt=6.58.

Example 41. $Q^4$=N-Methyl-N-(3-pyridylmethyl)amino; MS: m/z=567(M+1); HPLC: Solvent B, rt=6.74.

Example 42. $Q^4$=4-Pyridylmethylamino; MS: m/z=553(M+1); HPLC: Solvent B, rt=5.64.

Example 43. $Q^4$=3,5-Dimethylanilino; MS: m/z=566(M+1); HPLC: Solvent B, rt=20.55.

Example 44. $Q^4$=3,4-Dichlorobenzylamino; MS: m/z=624(M+1); HPLC: Solvent B, rt=16.75.

Example 45. $Q^4$=3-Methoxybenzylamino; MS: m/z=582(M+1); HPLC: Solvent B, rt=12.18.

Example 46. $Q^4$=2-Methoxyphenethylamino; MS: m/z=596,598(M+1); HPLC: Solvent B, rt=14.36.

Example 47. $Q^4$=4-Chlorophenethylamino; MS: m/z=602(M+1); HPLC: Solvent B, rt=18.64.

Example 48. $Q^4$=N-(Methyl)phenethylamino; MS: m/z=580(M+1); HPLC: Solvent B, rt=20.44.

Example 49. $Q^4$=3,4,5-Trimethoxyanilino; MS: m/z=628(M+1); HPLC: Solvent B, rt=10.67.

Example 50. $Q^4$=2-(1-Methylpyrrol-2-yl)ethylamino; MS: m/z=569(M+1); HPLC: Solvent B, rt=10.71.

Example 51. $Q^4$=2,6-Dichlorobenzylamino; MS: m/z=624(M+1); HPLC: Solvent B, rt=16.55.

Example 52. $Q^4$=N-Methyl-N-(2-pyrid-2-ylethyl)amino; MS: m/z=581(M+1); HPLC: Solvent B, rt=6.64.

Example 53. $Q^4$=3,4,5-Trimethoxybenzylamino; MS: m/z=642(M+1); HPLC: Solvent B, rt=9.57.

Example 54. $Q^4$=4-Aminobenzylamino; MS: m/z=567(M+1); HPLC: Solvent B, rt=5.63.

Examples 55-61

Using a procedure similar to that described in Example 16, except replacing the 1,2,3,4-tetrahydroisoquinoline with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined, were prepared.

Example 55. $Q^4$=N-(3,5-Dichlorobenzyl)-N-methylamino; MS: m/z=634(M+1).

Example 56. $Q^4$=4-(Ethoxycarbonyl)anilino; MS: m/z=610(M+1).

Example 57. $Q^4$=N-methyl-N-(3-methoxyphenethyl)amino; MS: m/z=610(M+1). Analysis for: $C_{34}H_{41}Cl_2N_3O_3$•1.0 HCL: Calculated: C, 60.58; H, 6.73; N, 6.23; Found: C, 60.40; H, 6.40; N, 6.63.

Example 58. $Q^4$=N-Indan-1-yl-N-methylamino; MS: m/z=592(M+1). Analysis for: $C_{34}H_{39}Cl_2N_3O_2$•0.65 $H_2O$: Calculated: C, 67.58; H, 6.72; N, 6.95; Found: C, 67.51; H, 6.63; N, 6.97.

Example 59. $Q^4$=N=Methyl-N-(2-methoxyphenethyl)-amino; MS: m/z=610(M+1). Analysis for: $C_{34}H_{41}Cl_2N_3O_3$•0.70 $H_2O$•1.0 HCl: Calculated: C, 61.90; H, 6.63; N, 6.37; Found: C, 61.84; H, 6.52; N, 6.41.

Example 60. $Q^4$=3-Hydroxy-3-phenylpiperidino; MS: m/z=622(M+1).

Example 61. $Q^4$=4-Phenylpiperidino; MS: m/z=621(M+1). Analysis for: $C_{36}H_{44}Cl_2N_3O_2$•1.30 $H_2O$•1.0 Iodide: Calculated: C, 56.01; H, 6.08; N, 5.44; Found: C, 55.89; H, 5.80; N, 5.35.

Examples 62-115

Using a procedure similar to that described in Example 16, except replacing the 1,2,3,4-tetrahydroisoquinoline with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined, were prepared.

Example 62. $Q^4$=Cyclopentylamino; MS: m/z=6530(M+1).

Example 63. $Q^4$=Cyclohexylamino; MS: m/z=544(M+1).

Example 64. $Q^4$=1,2,3,4-Tetrahydronaphth-1-ylamino; MS: m/z=592(M+1).

Example 65. $Q^4$=(1-Naphthylmethyl)amino; MS: m/z=602(M+1).

Example 66. $Q^4$=N,N-dibenzylamino; MS: m/z=642(M+1).

Example 67. $Q^4$=Thiazol-2-ylamino; MS: m/z=545(M+1).

Example 68. $Q^4$=(2-Indol-3-yl-1-methylethyl)amino; MS: m/z=619(M+1).

Example 69. $Q^4$=(2-Pyridylmethyl)amino; MS: m/z=553(M+1).

Example 70. $Q^4$=3-Trifluoromethylbenzylamino; MS: m/z=620(M+1).

Example 71. $Q^4$=3-Methylbenzylamino; MS: m/z=566(M+1).

Example 72. $Q^4$=3-(Carbamoyl)piperidino; MS: m/z=573(M+1).

Example 73. $Q^4$=(3,3-Dimethylbutyl)amino; MS: m/z=546(M+1).

Example 74. $Q^4$=N-Butyl-N-methylamino; MS: m/z=532(M+1).

Example 75. $Q^4$=2-Indanylamino; MS: m/z=578(M+1).

Example 76. $Q^4$=N-Methyl-N-(1-naphthylmethyl)amino; MS: m/z=616(M+1).

Example 77. $Q^4$=6,7-Dimethoxy-1,2,3,4-tetrahydroisoquinol-2-yl; MS: m/z=638(M+1).

Example 78. $Q^4$=$\alpha$-Oxophenethylamino; MS: m/z=580(M+1).

Example 79. $Q^4$=N-Ethyl-N-(4-pyridylmethyl)amino; MS: m/z=581(M+1).
Example 80. $Q^4$=N-[(R)-α-methylbenzyl]-N-methylamino; MS: m/z=580(M+1).
Example 81. $Q^4$=(R)-N-(1-Naphth-1-ylethyl)amino; MS: m/z=616(M+1).
Example 82. $Q^4$=(S)-N-(1-Naphth-1-ylethyl)amino; MS: m/z=616(M+1).
Example 83. $Q^4$=(R)-α-methylbenzylamino; MS: m/z=566(M+1).
Example 84. $Q^4$=(S)-α-methylbenzylamino; MS: m/z=566(M+1).
Example 85. $Q^4$=N-Methyl-N-[(R)-α-methylbenzyl]amino; MS: m/z=580(M+1).
Example 86. $Q^4$=N-(3,4-Dimethoxyphenethyl)-N-methylamino; MS: m/z=640(M+1).
Example 87. $Q^4$=N-(2-Indol-3-ylethyl)-N-methylamino; MS: m/z=619(M+1).
Example 88. $Q^4$=2-(5-Methoxyindol-3-yl)ethylamino; MS: m/z=635(M+1).
Example 89. $Q^4$=2-(6-Methoxyindol-3-yl)ethylamino; MS: m/z=635(M+1).
Example 90. $Q^4$=Benzothiazo-2-ylamino; MS: m/z=595(M+1).
Example 91. $Q^4$=2-Pyridylamino; MS: m/z=539(M+1).
Example 92. $Q^4$=(3-Pyridylmethyl)amino; MS: m/z=553(M+1).
Example 93. $Q^4$=2-Fluorobenzylamino; MS: m/z=570(M+1).
Example 94. $Q^4$=2,4-Dichlorobenzylamino; MS: m/z=621(M+1).
Example 95. $Q^4$=2-Ethozybenzylamino; MS: m/z=596(M+1).
Example 96. $Q^4$=3-Fluorobenzylamino; MS: m/z=570(M+1).
Example 97. $Q^4$=3,4-Dimethoxybenzylamino; MS: m/z=612(M+1).
Example 98. $Q^4$=4-Fluorobenzylamino; MS: m/z=570(M+1).
Example 99. $Q^4$=4-Methoxybenzylamino; MS: m/z=582(M+1).
Example 100. $Q^4$=4-Methylbenzylamino; MS: m/z=566(M+1).
Example 101. $Q^4$=(2-Methylbutyl)amino; MS: m/z=532(M+1).
Example 102. $Q^4$=Phenethylamino; MS: m/z=566(M+1).
Example 103. $Q^4$=2-Chlorophenethylamino; MS: m/z=601(M+1).
Example 104. $Q^4$=3-Methoxyphenethylamino; MS: m/z=596(M+1).
Example 105. $Q^4$=4-Bromophenethylamino; MS: m/z=644(M+1).
Example 106. $Q^4$=(3-Methylbutyl)amino; MS: m/z=532(M+1).
Example 107. $Q^4$=(butyl)(ethyl)amino; MS: m/z=546(M+1).
Example 108. $Q^4$=2,6-Difluorobenzylamino; MS: m/z=588(M+1).
Example 109. $Q^4$=4-Aminocarbonyl-4-(isopropylamino)piperidino; MS: m/z=630(M+1).
Example 110. $Q^4$=3,5-Dichlorobenzylamino; MS: m/z=620(M+1).
Example 111. $Q^4$=N-Benzyl-N-[(S)-a-methylbenzyl]amino; MS: m/z=656(M+1).
Example 112. $Q^4$=4-Aminocarbonyl-4-(methylamino)piperidino; MS: m/z=602(M+1).
Example 113. $Q^4$=;3-Methyl-3-phenylpiperidino MS: m/z=620(M+1).
Example 114. $Q^4$=(5-Methylfuran-2-ylmethyl)amino; MS: m/z=556(M+1).
Example 115. $Q^4$=(5-Methyl-1,3,4-thiadiazol-2-yl)amino; MS: m/z=560(M+1).

Examples 116-117

Using a procedure similar to that described in Example 16, except replacing the 1,2,3,4-tetrahydroisoquinoline with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined, were prepared..
Example 116. $Q^4$=N-Ethyl-N-(2-methoxybenzyl)amino; MS (CI): m/z=610(M+1). Analysis for $C_{34}H_{41}Cl_2N_3O_3$•1.O HCl•0.5$H_2O$: Calculated: C, 62.24; H, 6.60; N, 6.40; Found: C, 62.15; H, 6.55; N, 6.45.
Example 117. $Q^4$=N-(3,5-Dimethylbenzyl)-N-methylamino; MS (CI): m/z=594(M+1). Analysis for $C_{34}H_{41}Cl_2N_3O_2$•1.0 HCl•1.6 $H_2O$: Calculated: C, 61.88; H, 6.90; N, 6.37; Found: C, 61.77; H, 6.64; N, 6.10.

Example 118. 5-(4-Acetamido-4-phenylpiperidino)-3-(3,4-dichlorophenyl)-N-[3,5-bis(trifluoromethyl)benzyl] pentanamide.

Using a procedure similar to that described in Example 1, except replacing ethyl 5-(4-hydroxy-4-phenylpiperidino)-3-(3,4-dichlorophenyl)pentanoate with ethyl 5-(4-acetamido-4-phenyl-piperidino)-3-(3,4-dichlorophenyl)pentanoate and replacing benzyl amine with bis(trifluoromethyl)benzylamine, the title compound was prepared; mp 130-138 °C; MS: m/z=690(M+1); NMR (dimethylsulfoxide-d6): 1.93 (s,3), 2.0-2.18 (m,4), 2.50-2.70 (m,4), 2.79-2.94 (broad,1), 3.0-3.26 (m,4), 3.50 (t,2), 4.23-4.53 (m,2), 7.10-7.63 (m,8), 7.80 (s,2), 7.89 (s,1). Analysis for $C_{33}H_{33}Cl_2F_6N_3O_2$: Calculated: C, 57.57; H, 4.83; N, 6.10; Found: C, 57.62; H, 5.03; N, 5.94.

Examples 119-121

Using a procedure similar to that described in Example 16, except replacing the 1,2,3,4-tetrahydroisoquinoline with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined, were prepared..

Example 119. $Q^4$=N-(3,5-Dimethylbenzyl)amino; MS: m/z=580(M+1). Analysis for $C_{33}H_{39}Cl_2N_3O_2$•0.35 $H_2O$: Calculated: C, 67.49; H, 6.82; N, 7.73; Found: C, 67.48; H, 6.71; N, 7.06.

Example 120. $Q^4$=Anilino; MS: m/z=538(M+1). Analysis for $C_{30}H_{33}Cl_2N_3O_2$·0.30 $H_2O$; Calculated: C, 66.24; H, 6.23; N, 7.73; Found: C, 66.44; H, 6.17; N, 7.51.

Example 121. $Q^4$=3,5-Dichloroanilino; MS: m/z=606(M+1). Analysis for $C_{30}H_{31}Cl_4N_3O_2$•0.23 $H_2O$: Calculate: C, 58.92; H, 5.18; N, 6.87; Found: C, 58.91; H, 5.20; N, 6.94.

Example 122. (R*)-5-(4-Acetamido-4-phenylpiperidino)-3-(3,4-dichlorophenyl)-N-(2-methoxybenzyl)-N-methylpentanamide.

Using a procedure similar to that described in Example 16, except replacing the 1,2,3,4-tetrahydroisoquinoline with 4-acetamido-4-phenylpiperidine, and replacing the racemic 5-(4-acetamido-4-phenylpiperidino)-3-(3,4-dichlorophenyl)pentanoic acid with the requsite enantiomer, the title compound was prepared; MS: m/z=596(M+1). Analysis for $C_{33}H_{39}Cl_2N_3O_3$•0.30 $H_2O$: Calculated: C, 65.84; H, 6.63; N, 6.98; Found: C, 65.74; H, 6.58; N, 7.10.

Example 123. (S*)-5-(4-Acetamido-4-phenylpiperidino)-3-(3,4-dichlorophenyl)-N-(2-methoxybenzyl)-N-methylpentanamide

Using a procedure similar to that described in Example 16, except replacing the 1,2,3,4-tetrahydroisoquinoline with 4-acetamido-4-phenylpiperidine, and replacing the racemic 5-(4-acetamido-4-phenylpiperidino)-3-(3,4-dichlorophenyl)pentanoic acid with the requsite enantiomer, the title compound was prepared; MS: m/z=596(M+1). Analysis for $C_{33}H_{39}Cl_2N_3O_3$•0.2 $H_2O$: Calculated: C, 66.04; H, 6.62: N, 7.00; found: C, 66.04; H, 6.67; N, 7.16.

The intermediate chiral acid used for the preparation of the compounds of Examples 122 and 123 were prepared as follows. These acids are also compounds of the invention.

A racemic mixture of the ethyl ester of Example 15 was resolved by high performance liquid chromatogyaphy using a Chiracel OD (50mm x 50 cm ) column, with hexane:ethanol (50:50) as the eluent and a flow rate of 54 mL/minute. The first ester enantiomer to elute had a retention time of 14 minutes. This ester was hydrolysed under standard conditions to give the corresponding acid. The acid was used for the coupling reaction in Example 122. The second ester enantiomer to elute had a retention time of 19 minutes. This ester was hydrolysed under standard conditions to give the corresponding acid. The acid was used for the coupling reaction in Example 123.

Reference Example A. 5-[4-(2-Oxoperhydropyrimidin-1-yl)piperidino]-3-(3,4-dichlorophenyl)-N-(2-methoxybenzyl)-N-methylpentanamide.

3-(3,4-Dichlorophenyl)-N-(2-methoxybenzyl)-N-methyl-5-oxopentanamide (0.1 molar in tetrahydrofuran, 5 mL) was added through a syringe to 2-oxoperhydropyrimidin-1-ylpiperidine (0.5 mmol) and the mixture was pulse-vortexed for 30 seconds. Glacial acetic acid (0.7 mmol) was added and the solution was allowed to stir for 10 minutes at room temperature. A solution of sodium cyanoborohydride in methanol (0.08 molar, 10 mL) was added in 4 portions with pulse-vortexing between each addition. The reaction was allowed to stir over night and the volume was decreased to approximately 3 mL using a centrifugal evaporator. Ethyl acetate was added and the soulution was pulse-vortexed for for 30 seconds. The solution was taken up using a canula and transfered to a 25x150 mm tube. Ethyl acetate (2 mL) and saturated aqueous sodium bicarbonate (9 mL) were added and the mixture was pulse-vortexed. After 20 minutes, the aqueous layer was removed and the organic layer was washed with brine (8 mL). After 20 minutes, the brine was removed and the organig solution was concentrated using a centrifugal evaporator. The concentrate was transfered to vials and evaporated to give the title compound; MS: m/z=561(M+1). Analysis for $C_{29}H_{38}Cl_2N_4O_3$•0.50 $H_20$: Calculated: C, 61.05; H, 6.89; N, 9.82; Found: C, 61.04; H, 6.73; N, 9.73. The procedure may conveniently be carried out with the assistance of a robot.

Example 124. 3-(3,4-Dichlorophenyl)-5-(4-hydroxy-4-phenylpiperidino)-N-methyl-N-(2-methylphenyl)pentanamide.

Using a procedure similar to that described in Example 1, except replacing the benzylamine used therein with N-methyl-N-(2-methylphenyl)amine, the title compound was prepared; MS: m/z=509(M+1).

Example 125. 4-Acetamido-1-[3-(3,4-dichlorophenyl)-4-(N-(2-methoxybenzyl)-N-methylaminocarbonylbutyl]-1-methyl-4-phenylpiperidinium iodide.

N-Methyl-N-2-(methoxybenzyl)-5-(4-acetamido-4-phenylpiperidino)-3-(3,4-dichlorophenyl)pentanamide (the compound of Example 10, 0.075 g) was dissolved in dichloromethane (5 mL). Iodomethane (71 mg) was added. After 2 days at room temperature, the solution was evaporated. The resulting residue was triturated with ethyl ether to give the title compound (0.070 g); MS: m/z=611(M+1). Analysis for $C_{34}H_{42}Cl_2IN_3O_3$•0.65 $H_2O$: Calculated: C, 54.43; H, 5.82; N, 5.60; Found: C, 54.76; H, 5.79; N, 5.43.

Example 126. 4-Acetamido-1-[3-(3,4-dichlorophenyl)-4-(N-(2-methoxybenzyl)-N-methylaminocarbonylbutyl]-4-phenylpiperidine 1-oxide.

N-Methyl-N-2-(methoxybenzyl)-5-(4-acetamido-4-phenylpiperidino)-3-(3,4-dichlorophenyl)pentanamide (the compound of Example 10, 0.100 g) was dissolved in 5 mL dichloromethane and treated with meta-chloroperoxybenzoic acid (0.100 g) at room temperature. The mixture was stirred for 3 hours, evaporated and purified by column chromatography on neutral alumina, eluting first with dichloromethane and gradually increasing to 9:1 dichloromethane: methanol, to give the title compound (58 mg) as a foamy solid; MS: m/z=612(M+1). Analysis for $C_{33}H_{39}Cl_2N_3O_4$•1.30 $H_2O$: Calculated: C, 62.32; H, 6.58; N, 6.61; Found: C, 62.12; H, 6.21; N, 6.71.

Examples 127

Using a procedure similar to that described in Reference Example A, except replacing the 2-oxoperhydropyrimidin-1-ylpiperidine with the requisite piperidine, the following compounds of formula I wherein $Q^2$ is 3,4-dichlorophenyl, $Q^3$ is hydrogen, $Q^4$ is N-(2-methoxybenzyl)-N-methylamino, and $Q^1$ is as defined were prepared.
Example 127. $Q^1$=4-Hydroxy-4-phenylpiperidino; MS: m/z=555(M+1).

Example 128. N-Benzyl-5-(4-hydroxy-4-phenylpiperidino)-3-(3,4-dichlorophenyl)-N-methylpentanamide hydrochloride salt.

Using a procedure similar to that described in Example 1, except replacing the benzylamine used therein with N-benzyl-N-methylamine followed by conversion of the free amine to the hydrochloride salt as described in Example 2, the title compound was prepared; MS: m/z=525(M+1). Analysis for $C_{30}H_{34}Cl_2N_2O_2$•0.5 $H_20$•1.0 HCl: Calculated: C, 63.10;

Examples 129 and 130

Using a procedure similar to that described in Example 10, except replacing the N-methyl-N-(2-methoxybenzyl)amine with the requisite amine, the following compounds of formula I wherein $Q^1$ is 4-acetamido-4-phenylpiperidino, $Q^2$ is 3,4-dichlorophenyl, $Q^3$ is hydrogen and $Q^4$ is as defined were prepared.
Example 129. $Q^4$=N-(3,5-dichloro-2-methoxybenzyl)-N-methylamino; MS: m/z=665(M+1).
Example 130. $Q^4$=N-(3,5-dichloro-2-methoxybenzyl)amino; MS: m/z=651(M+1).

Examples 131-132

Using procedures similar to those described hereinabove, the following compounds of formula I wherein $Q^2$ is 3,4-dichlorophenyl, $Q^3$ is hydrogen, $Q^4$ is 1,2,3,4-tetrahydronaphth-1-ylamino, and $Q^1$ is as defined can be prepared.
Example 131. $Q^1$=4-Hydroxy-4-phenylpiperidino.
Example 132. $Q^1$=4-Acetamido-4-phenylpiperidino.

Examples 133-134

Using procedures similar to those described hereinabove, the following compounds of formula I wherein $Q^2$ is 3,4-dichlorophenyl, $Q^3$ is hydrogen, $Q^4$ is 2-fluorobenzylamino, and $Q^1$ is as defined can be prepared.
Example 133. $Q^1$=4-Hydroxy-4-phenylpiperidino.
Example 134. $Q^1$ = 4-Acetamido-4-phenylpiperidino.

## Claims

**1.** A compound of formula I:

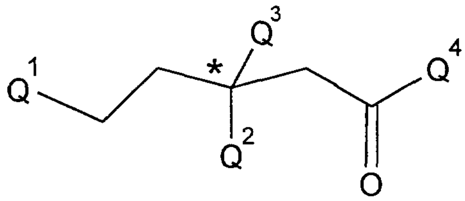

I

wherein

$Q^1$ is 4-hydroxy-4-phenylpiperidino or 4-acetamido-4-phenylpiperidino;
$Q^2$ is phenyl which may bear one or two substituents independently selected from halo, trifluoromethyl, hydroxy, (1-3C)alkoxy, (1-3C)alkyl and methylenedioxy; or $Q^2$ is thienyl, imidazolyl, benzo[b]thiophenyl or naphthyl any of which may bear a halo substituent; or $Q^2$ is biphenylyl; or $Q^2$ is carbon-linked indolyl which may bear a benzyl substituent at the 1-position;
$Q^3$ is hydrogen, or (1-4C)alkyl; and
$Q^4$ is $-OR^2$ or $-NR^3R^4$; wherein
$R^2$ is hydrogen, (1-6C)alkyl, (3-7C)cycloalkyl, aryl(1-3C)alkyl or heteroaryl(1-3C)alkyl, wherein an aryl or heteroaryl group may bear one, two or three substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, and methylenedioxy, and further wherein any arylethyl, arylpropyl, heteroarylethyl or heteroarylpropyl group may optionally be substituted at the position $\alpha$ to the aryl or heteroaryl group by a group selected from oxo, and $=NOR^{11}$;
$R^3$ and $R^4$ are independently selected from hydrogen, (1-6C)alkyl, (3-7C)cycloalkyl, aryl, heteroaryl, aryl(1-3C) alkyl and heteroaryl(1-3C)alkyl, wherein any aryl or heteroaryl group may bear one two or three substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, and methylenedioxy, and further wherein any arylethyl, arylpropyl, heteroarylethyl or heteroarylpropyl group may optionally be substituted at the position $\alpha$ to the aryl or heteroaryl group by a group selected from oxo, and $=NOR^{11}$; or
$-NR^3R^4$ taken together represents a cyclic amino radical selected from pyrrolidinyl, piperidino, 1,2,3,6-tetrahydropyridyl, 1,2,3,4-tetrahydroquinolyl, and 1,2,3,4-tetrahydroisoquinolyl, which cyclic amino radical may bear one or two substituents independently selected from halo, trifluoromethyl, hydroxy, (1-3C)alkoxy, (1-3C)alkyl, cyano, $-S(=O)^2NR^5R^6$, $-NR^7R^8$, $C(=O)NR^9R^{10}$, phenyl, acetamidomethyl, and methylenedioxy; and
$R^5$-$R^{11}$ are independently selected from hydrogen and (1-3C)alkyl; and
wherein the term aryl denotes a phenyl radical or an ortho-fused bicyclic carbocyclic radical having nine to ten ring atoms in which at least one ring is aromatic and the term heteroaryl means a radical of a monocyclic aromatic ring containing five ring atoms, consisting of carbon and one to four heteroatoms selected from oxygen, sulfur and nitrogen or containing six ring atoms consisting of carbon and one or two nitrogens, as well as a radical of an ortho-fused bicyclic heterocycle of eight to ten atoms derived therefrom, or a stable N-oxide thereof;
or the N-oxide of the piperidino nitrogen in $Q^1$;
or a pharmaceutically acceptable salt thereof;
or a quaternary ammonium salt thereof in which the piperidino nitrogen in $Q^1$ is a quadricovalent ammonium nitrogen wherein the fourth radical on the nitrogen $R^1$ is (1-4C)alkyl or benzyl and the associated counterion A is a pharmaceutically acceptable anion.

**2.** A compound as claimed in claim 1 wherein:

$Q^3$ is hydrogen;
$Q^4$ is $-OR^2$ or $-NR^3R^4$; wherein
$R^2$ is hydrogen, (1-6C)alkyl, (3-7C)cycloalkyl, aryl(1-3C)alkyl or heteroaryl(1-3C)alkyl, wherein an aryl or heteroaryl group may bear one, two or three substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, and methylenedioxy;
$R^3$ and $R^4$ are independently selected from hydrogen, (1-6C)alkyl, (3-7C)cycloalkyl, aryl, heteroaryl, aryl(1-3C)alkyl and heteroaryl(1-3C)alkyl, wherein any aryl or heteroaryl group may bear one two or three substituents independently selected from halo, trifluoromethyl, hydroxy, (1-3C)alkoxy, (1-3C)alkyl, cyano, $-S(=O)_2NR^5R^6$, $-NR^7R^8$, $C(=O)NR^9R^{10}$, and methylenedioxy; or
$-NR^3R^4$ taken together represents a cyclic amino radical selected from pyrrolidinyl, piperidino, 1,2,3,6-tetrahydropyridyl, 1,2,3,4-tetrahydroquinolyl, and 1,2,3,4-tetrahydroisoquinolyl, which cyclic amino radical may bear one or two substituents independently selected from halo, trifluoromethyl, hydroxy, (1-3C)alkoxy, (1-3C)alkyl, cyano, $-S(=O)^2NR^5R^6$, $-NR^7R^8$, $C(=O)NR^9R^{10}$, phenyl, acetamidomethyl, and methylenedioxy.

**3.** A compound as claimed in any preceding claim wherein $Q^2$ is 3,4-dichlorophenyl or 3,4-methylenedioxyphenyl.

**4.** A compound as claimed in any preceding claim wherein $Q^2$ is 3,4-dichlorophenyl.

**5.** A compound as claimed in any preceding claim wherein $Q^4$ is $-NR^3R^4$

**6.** A compound as claimed in any preceding claim wherein $Q^4$ is (2-methoxybenzyl)(methyl)amino

**7.** A pharmaceutical composition comprising a compound of formula I;

or the N-oxide of the piperidino nitrogen in $Q^1$;
or a pharmaceutically acceptable salt thereof;
or a quaternary ammonium salt thereof in which the piperidino nitrogen in $Q^1$ is a quadricovalent ammonium nitrogen wherein the fourth radical on the nitrogen $R^1$ is (1-4C)alkyl or benzyl and the associated counterion A is a pharmaceutically acceptable anion; as defined in any one of claims 1-6; and a pharmaceutically acceptable diluent or carrier.

**8.** A process for the manufacture of a compound of formula I: or the N-oxide of the piperidino nitrogen in $Q^1$; or a pharmaceutically acceptable salt thereof; or a quaternary ammonium salt thereof in which the piperidino nitrogen in $Q^1$ is a quadricovalent ammonium nitrogen wherein the fourth radical on the nitrogen $R^1$ is (1-4C)alkyl or benzyl and the associated counterion A is a pharmaceutically acceptable anion; as defined in any one of claims 1-6, which is characterized by:

(a) Acylating an amine of formula $-NR^3R^4$, with an ester of formula IV:

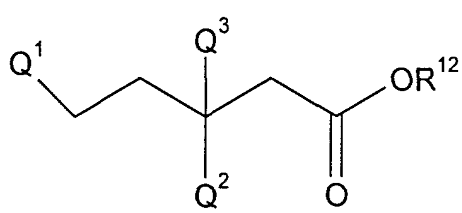

IV

wherein $R^{12}$ is a suitable alkyl radical such as for example (1-3C)alkyl;
(b) For an acid addition salt of a compound of formula I, treating a corresponding compound of formula I which is in the free-base form, with an acid
(c) Alkylating an amine of formula $Q^1$-H with an aldehyde of formula V:

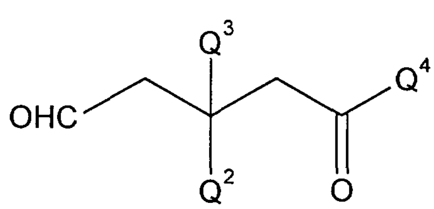

V

by reductive alkylation;
(d) Acylating an amine of formula $-NR^3R^4$, with an acid of formula IV:

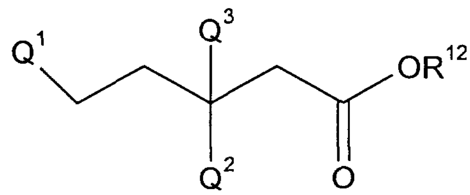

IV

wherein $R^{12}$ is a hydrogen;

(e) Alkylating an amine of formula $Q^1$-H with an alkylating agent of formula VI:

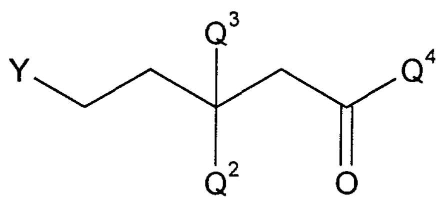

VI

in which Y is a conventional leaving group;

(f) For an N-oxide of the piperidino nitrogen in $Q^1$; oxidizing the nitrogen of a corresponding compound of formula I using a conventional procedure;

(g) For a quaternary ammonium salt of the piperidino nitrogen in $Q^1$, alkylating the nitrogen in a corresponding compound of formula I with an alkylating agent of formula $R^1Y$ wherein Y is a leaving group;

(j) For a compound of formula I which bears an aromatic hydroxy group, cleaving the ether of a corresponding compound of formula I which bears an aromatic alkoxy group.

**Patentansprüche**

**1.** Verbindung der Formel I:

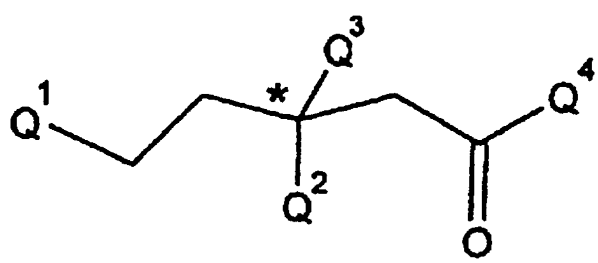

I

worin

$Q^1$ für 4-Hydroxy-4-phenylpiperidino oder 4-Acetamido-4-phenylpiperidino steht;

$Q^2$ für gegebenenfalls einen oder zwei unabhängig voneinander unter Halogen, Trifluormethyl, Hydroxy, $C_{1-3}$-Alkoxy, $C_{1-3}$-Alkyl und Methylendioxy ausgewählte Substituenten tragendes Phenyl; Thienyl, Imidazolyl, Benzo [b] thiophenyl oder Naphthyl, die jeweils einen Halogensubstituenten tragen können; Biphenylyl oder über Kohlenstoff verknüpftes Indolyl, das in 1-Stellung einen Benzylsubstituenten tragen kann, steht;

$Q^3$ für Wasserstoff oder $C_{1-4}$-Alkyl steht;

$Q^4$ für $-OR^2$ oder $-NR^3R^4$ steht; worin

$R^2$ für Wasserstoff, $C_{1-6}$-Alkyl, $C_{3-7}$-Cycloalkyl, Aryl-$C_{1-3}$-alkyl oder Heteroaryl-$C_{1-3}$-alkyl, worin eine Aryl- oder Heteroarylgruppe einen, zwei oder drei unabhängig voneinander unter Halogen, Trifluormethyl, Hydroxy, $C_{1-3}$-Alkoxy, $C_{1-3}$-Alkyl, Cyano, $-S(=O)_2NR^5R^6$, $-NR^7R^8$, $C(=O)NR^9R^{10}$ und Methylendioxy ausgewählte Substituenten tragen kann und ferner jede Arylethyl-, Arylpropyl-, Heteroarylethyl- oder Heteroarylpropylgruppe gegebenenfalls in α-Stellung zur Aryl- bzw,. Heteroarylgruppe durch eine unter Oxo und =$NOR^{11}$ ausgewählte Gruppe substituiert sein kann, steht;
$R^3$ und $R^4$ unabhängig voneinander unter Wasserstoff, $C_{1-6}$-Alkyl, $C_{3-7}$-Cycloalkyl, Aryl, Heteroaryl, Aryl-$C_{1-3}$-alkyl und Heteroaryl-$C_{1-3}$-alkyl, worin jede Aryl- oder Heteroarylgruppe einen, zwei oder drei unabhängig voneinander unter Halogen, Trifluormethyl, Hydroxy, $C_{1-3}$-Alkoxy, $C_{1-3}$-Alkyl, Cyano, -$S(=O)_2NR^5R^6$, -$NR^7R^8$, C(=O)$NR^9R^{10}$ und Methylendioxy ausgewählte Substituenten tragen kann und ferner jede Arylethyl-, Arylpropyl-, Heteroarylethyl- oder Heteroarylpropylgruppe gegebenenfalls in α-Stellung zur Aryl- bzw,. Heteroarylgruppe durch eine unter Oxo und =$NOR^{11}$ ausgewählte Gruppe substituiert sein kann, ausgewählt sind; oder
-$NR^3R^4$ zusammengenommen für einen unter Pyrrolidinyl, Piperidino, 1,2,3,6-Tetrahydropyridyl, 1,2,3,4-Tetrahydrochinolyl und 1,2,3,4-Tetrahydroisochinolyl ausgewählten cyclischen Aminorest, welcher einen oder zwei unabhängig voneinander unter Halogen, Trifluormethyl, Hydroxy, $C_{1-3}$-Alkoxy, $C_{1-3}$-Alkyl, Cyano, -S(=O)$_2NR^5R^6$, -$NR^7R^8$, C(=O) $NR^9R^{10}$, Phenyl, Acetamidomethyl und Methylendioxy ausgewählte Substituenten tragen kann, steht; und
$R^5$-$R^{11}$ unabhängig voneinander unter Wasserstoff und $C_{1-3}$-Alkyl ausgewählt sind; und
worin der Begriff Aryl einen Phenylrest oder einen ortho-anellierten bicyclischen carbocyclischen Rest mit neun bis zehn Ringatomen, wobei mindestens ein Ring aromatisch ist, und der Begriff Heteroaryl einen Rest eines monocyclischen aromatischen Rings mit fünf Ringatomen, bestehend aus Kohlenstoff und eins bis vier unter Sauerstoff, Schwefel und Stickstoff ausgewählten Heteroatomen oder mit sechs Ringatomen, bestehend aus Kohlenstoff und einem oder zwei Stickstoffatomen, sowie einen davon abgeleiteten Rest eines ortho-anellierten bicyclischen Heterocyclus mit acht bis zehn Atomen oder ein stabiles N-Oxid davon bezeichnet;
oder das N-Oxid des Piperidinstickstoffs in $Q^1$;
oder ein pharmazeutisch unbedenkliches Salz davon;
oder ein quartäres Ammoniumsalz davon, in dem der Piperidinstickstoff in $Q^1$ ein vierwertiger Ammoniumstickstoff ist, worin der vierte Rest $R^1$ am Stickstoff für $C_{1-4}$-Alkyl oder Benzyl steht und das zugehörige Gegenion A ein pharmazeutisch unbedenkliches Anion darstellt.

**2.** Verbindung nach Anspruch 1, worin:

$Q^3$ für Wasserstoff steht;
$Q^4$ für -$OR^2$ oder -$NR^3R^4$ steht; worin
$R^2$ für Wasserstoff, $C_{1-6}$-Alkyl, $C_{3-7}$-Cycloalkyl, Aryl-$C_{1-3}$-alkyl oder Heteroaryl-$C_{1-3}$-alkyl, worin eine Aryl- oder Heteroarylgruppe einen, zwei oder drei unabhängig voneinander unter Halogen, Trifluormethyl, Hydroxy, $C_{1-3}$-Alkoxy, $C_{1-3}$-Alkyl, Cyano, -S(=O)$_2NR^5R^6$, -$NR^7R^8$, C(=O)$NR^9R^{10}$ und Methylendioxy ausgewählte Substituenten tragen kann, steht;
$R^3$ und $R^4$ unabhängig voneinander unter Wasserstoff, $C_{1-6}$-Alkyl, $C_{3-7}$-Cycloalkyl, Aryl, Heteroaryl, Aryl-$C_{1-3}$-alkyl und Heteroaryl-$C_{1-3}$-alkyl, worin jede Aryl- oder Heteroarylgruppe einen, zwei oder drei unabhängig voneinander unter Halogen, Trifluormethyl, Hydroxy, $C_{1-3}$-Alkoxy, $C_{1-3}$-Alkyl, Cyano, -S(=O)$_2NR^5R^6$, -$NR^7R^8$, C(=O)$NR^9R^{10}$ und Methylendioxy ausgewählte Substituenten tragen kann, ausgewählt sind; oder
-$NR^3R^4$ zusammengenommen für einen unter Pyrrolidinyl, Piperidino, 1,2,3,6-Tetrahydropyridyl, 1,2,3,4-Tetrahydrochinolyl und 1,2,3,4-Tetrahydroisochinolyl ausgewählten cyclischen Aminorest, welcher einen oder zwei unabhängig voneinander unter Halogen, Trifluormethyl, Hydroxy, $C_{1-3}$-Alkoxy, $C_{1-3}$-Alkyl, Cyano, -S(=O)$_2NR^5R^6$, -$NR^7R^8$, C(=O)$NR^9R^{10}$, Phenyl, Acetamidomethyl und Methylendioxy ausgewählte Substituenten tragen kann, steht.

**3.** Verbindung nach einem der vorhergehenden Ansprüche, worin $Q^2$ für 3,4-Dichlorphenyl oder 3,4-Methylendioxyphenyl steht.

**4.** Verbindung nach einem der vorhergehenden Ansprüche, worin $Q^2$ für 3,4-Dichlorphenyl steht.

**5.** Verbindung nach einem der vorhergehenden Ansprüche, worin $Q^4$ für -$NR^3R^4$ steht.

**6.** Verbindung nach einem der vorhergehenden Ansprüche, worin $Q^4$ für (2-Methoxybenzyl)(methyl) amino steht.

**7.** Pharmazeutische Zusammensetzung, enthaltend eine Verbindung der Formel I;

oder das N-Oxid des Piperidinstickstoffs in $Q^1$;
oder ein pharmazeutisch unbedenkliches Salz davon;
oder ein quartäres Ammoniumsalz davon, in dem der Piperidinstickstoff in $Q^1$ ein vierwertiger Ammonium-

stickstoff ist, worin der vierte Rest $R^1$ am Stickstoff für $C_{1-4}$-Alkyl oder Benzyl steht und das zugehörige Gegenion A ein pharmazeutisch unbedenkliches Anion darstellt, gemäß einem der Ansprüche 1-6 und ein pharmazeutisch unbedenkliches Verdünnungsmittel oder einen pharmazeutisch unbedenklichen Träger.

**8.** Verfahren zur Herstellung einer Verbindung der Formel I; oder des N-Oxids des Piperidinstickstoffs in $Q^1$; oder eines pharmazeutisch unbedenklichen Salzes davon; oder eines quartären Ammoniumsalzes davon, in dem der Piperidinstickstoff in $Q^1$ ein vierwertiger Ammoniumstickstoff ist, worin der vierte Rest $R^1$ am Stickstoff für $C_{1-4}$-Alkyl oder Benzyl steht und das zugehörige Gegenion A ein pharmazeutisch unbedenkliches Anion darstellt, gemäß einem der Ansprüche 1-6, dadurch gekennzeichnet, daß man:

(a) ein Amin der Formel $-NR^3R^4$ mit einem Ester der Formel IV:

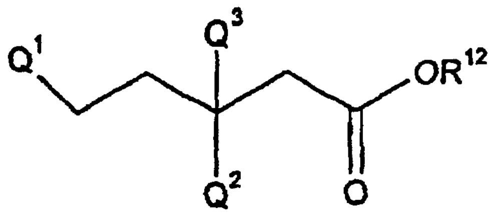

IV

worin $R^{12}$ für einen geeigneten Alkylrest wie beispielsweise $C_{1-3}$-Alkyl steht, acyliert;

(b) zur Herstellung eines Säureadditionssalzes einer Verbindung der Formel I eine entsprechende Verbindung der Formel I, die in Form der freien Base vorliegt, mit einer Säure behandelt;

(c) ein Amin der Formel $Q^1$-H mit einem Aldehyd der Formel V:

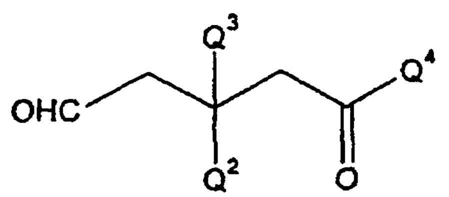

V

durch reduktive Alkylierung alkyliert;

(d) ein Amin der Formel $-NR^3R^4$ mit einer Säure der Formel IV:

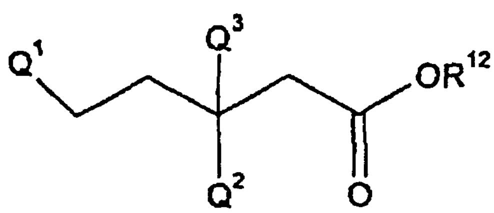

IV

worin $R^{12}$ für Wasserstoff steht, acyliert;

(e) ein Amin der Formel $Q^1$-H mit einem Alkylierungsmittel der Formel VI:

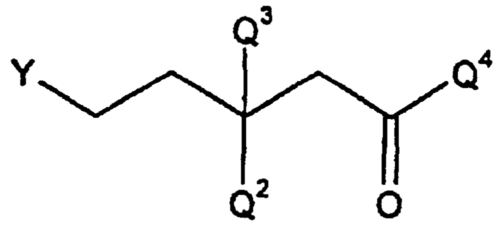

VI

worin Y für eine übliche Abgangsgruppe steht, alkyliert;

(f) zur Herstellung eines N-Oxids des Piperidinstickstoffs in $Q^1$ das Stickstoffatom einer entsprechenden Verbindung der Formel I nach einer herkömmlichen Verfahrensweise oxidiert;

(g) zur Herstellung eines quartären Ammoniumsalzes des Piperidinstickstoffs in $Q^1$ das Stickstoffatom in einer entsprechenden Verbindung der Formel I mit einem Alkylierungsmittel der Formel $R^1Y$, worin Y für eine Abgangsgruppe steht, alkyliert;

(j) zur Herstellung einer eine aromatische Hydroxylgruppe tragenden Verbindung der Formel I den Ether einer entsprechenden, eine aromatische Alkoxygruppe tragenden Verbindung der Formel I spaltet.

**Revendications**

**1.** Composé de formule I :

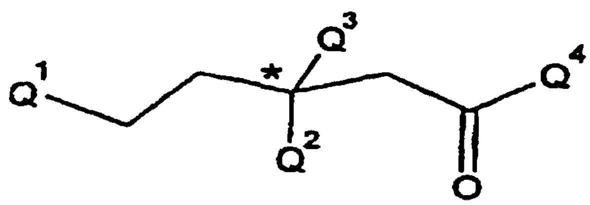

I

dans laquelle

$Q^1$ est un 4-hydroxy-4-phénylpipéridino ou un 4-acétamido-4-phénylpipéridino ;

$Q^2$ est un phényle pouvant renfermer un ou deux substituants choisis indépendamment parmi un halogéno, un trifluorométhyle, un hydroxy, un alcoxy en $C_{1-3}$, un alkyle en $C_{1-3}$ et un méthylènedioxy ; ou $Q^2$ est un thiényle, un imidazolyle, un benzo[b]thiophényle ou un naphtyle, chacun pouvant renfermer un substituant halogéno ; ou $Q^2$ est un biphényle ; ou $Q^2$ est un indolyle lié à un carbone pouvant renfermer un substituant benzyle en position 1 ;

$Q^3$ est un hydrogène ou un alkyle en $C_{1-4}$ ; et

$Q^4$ est $-OR^2$ ou $-NR^3R^4$ ; où

$R^2$ est un hydrogène, un alkyle en $C_{1-6}$, un cycloalkyle en $C_{3-7}$, un aryl-$C_{1-3}$-alkyle ou un hétéroaryl-$C_{1-3}$-alkyle, où un groupe aryle ou hétéroaryle peut renfermer un, deux ou trois substituants choisis indépendamment parmi un halogéno, un trifluorométhyle, un hydroxy, un alcoxy en $C_{1-3}$, un alkyle en $C_{1-3}$, un cyano, $-S(=O)_2NR^5R^6$, $-NR^7R^8$, $C(=O)NR^9R^{10}$, et un méthylènedioxy, et où, en outre, tout groupe aryléthyle, arylpropyle, hétéroaryléthyle ou hétéroarylpropyle peut être éventuellement substitué en position $\alpha$ par rapport au groupe aryle ou hétéroaryle par un groupe choisi parmi un oxo et $=NOR^{11}$ ;

$R^3$ et $R^4$ sont choisis indépendamment parmi un hydrogène, un alkyle en $C_{1-6}$, un cycloalkyle en $C_{3-7}$, un aryle, un hétéroaryle, un aryl-$C_{1-3}$-alkyle et un hétéroaryl-$C_{1-3}$-alkyle, où tout groupe aryle ou hétéroaryle peut renfermer un, deux ou trois substituants choisis indépendamment parmi un halogéno, un trifluorométhyle, un hydroxy, un alcoxy en $C_{1-3}$, un alkyle en $C_{1-3}$, un cyano, $-S(=O)_2NR^5R^6$, $-NR^7R^8$, $C(=O)NR^9R^{10}$, et un méthylènedioxy, et où, en outre, tout groupe aryléthyle, arylpropyle, hétéroaryléthyle ou hétéroarylpropyle peut être éventuellement substitué en position $\alpha$ par rapport au groupe aryle ou hétéroaryle par un groupe choisi parmi un oxo et $=NOR^{11}$; ou

$-NR^3R^4$, pris conjointement, représentent un radical aminocyclique choisi parmi une pyrrolidine, un pipéridino, un 1,2,3,6-tétrahydropyridyle, un 1,2,3,4-tétrahydroquinoléyle, et un 1,2,3,4-tétrahydroisoquinoléyle, lequel

radical aminocyclique peut renfermer un ou deux substituants choisis indépendamment parmi un halogéno, un trifluorométhyle, un hydroxy, un alcoxy en $C_{1-3}$, un alkyle en $C_{1-3}$, un cyano, -S $(=O)_2NR^5R^6$, $-NR^7R^8$, C (=O) $NR^9R^{10}$, un phényle, un acétamidométhyle et un méthylènedioxy ; et

$R^5$ à $R^{11}$ sont choisis indépendamment parmi un hydrogène et un alkyle en $C_{1-3}$ ; et

dans laquelle le terme aryle désigne un radical phényle ou un radical carbocyclique bicyclique condensé en ortho ayant neuf à dix atomes de noyau, dans lequel au moins un noyau est aromatique, et le terme hétéroaryle signifie un radical d'un noyau aromatique monocyclique renfermant cinq atomes de noyau, constitués de carbone et d'un à quatre hétéroatomes choisis parmi l'oxygène, le soufre et l'azote, ou renfermant six atomes de noyau constitués de carbone et d'un ou deux azotes, ainsi qu'un radical d'un hétérocycle bicyclique condensé en ortho ayant huit à dix atomes qui en est dérivé, ou un N-oxyde stable de celui-ci ;

ou le N-oxyde de l'azote de pipéridino dans $Q^1$;

ou un sel pharmaceutiquement acceptable de celui-ci ;

ou un sel d'ammonium quaternaire de celui-ci dans lequel l'azote de pipéridino dans $Q^1$ est un azote d'ammonium quadricovalent dans lequel le quatrième radical sur l'azote $R^1$ est un alkyle en $C_{1-4}$ ou un benzyle, et le contre-ion A associé est un anion pharmaceutiquement acceptable.

**2.** Composé selon la revendication 1, dans lequel :

$Q^3$ est un hydrogène ;

$Q^4$ est $-OR^2$ ou $-NR^3R^4$ ; où

$R^2$ est un hydrogène, un alkyle en $C_{1-6}$, un cycloalkyle en $C_{3-7}$, un aryl-$C_{1-3}$-alkyle ou un hétéroaryl-$C_{1-3}$-alkyle, où un groupe aryle ou hétéroaryle peut renfermer un, deux ou trois substituants choisis indépendamment parmi un halogéno, un trifluorométhyle, un hydroxy, un alcoxy en $C_{1-3}$, un alkyle en $C_{1-3}$, un cyano, -S $(=O)_2NR^5R^6$, $-NR^7R^8$, C (=O) $NR^9R^{10}$, et un méthylènedioxy ;

$R^3$ et $R^4$ sont choisis indépendamment parmi un hydrogène, un alkyle en $C_{1-6}$, un cycloalkyle en $C_{3-7}$, un aryle, un hétéroaryle, un aryl-$C_{1-3}$-alkyle et un hétéroaryl-$C_{1-3}$-alkyle, où tout groupe aryle ou hétéroaryle peut renfermer un, deux ou trois substituants choisis indépendamment parmi un halogéno, un trifluorométhyle, un hydroxy, un alcoxy en $C_{1-3}$, un alkyle en $C_{1-3}$, un cyano, $-S(=O)_2NR^5R^6$, $-NR^7R^8$, $C(=O)NR^9R^{10}$, et un méthylènedioxy ; ou

$-NR^3R^4$, pris conjointement, représentent un radical aminocyclique choisi parmi une pyrrolidine, un pipéridino, un 1,2,3,6-tétrahydropyridyle, un 1,2,3,4-tétrahydroquinoléyle, et un 1,2,3,4-tétrahydroisoquinoléyle, lequel radical aminocyclique peut renfermer un ou deux substituants choisis indépendamment parmi un halogéno, un trifluorométhyle, un hydroxy, un alcoxy en $C_{1-3}$, un alkyle en $C_{1-3}$, un cyano, -S $(=O)_2NR^5R^6$, $-NR^7R^8$, C (=O) $NR^9R^{10}$, un phényle, un acétamidométhyle et un méthylènedioxy.

**3.** Composé selon l'une quelconque des revendications précédentes, dans lequel $Q^2$ est un 3,4-dichlorophényle ou un 3,4-méthylènedioxyphényle.

**4.** Composé selon l'une quelconque des revendications précédentes, dans lequel $Q^2$ est un 3,4-dichlorophényle.

**5.** Composé selon l'une quelconque des revendications précédentes, dans lequel $Q^4$ est $-NR^3R^4$.

**6.** Composé selon l'une quelconque des revendications précédentes, dans lequel $Q^4$ est un (2-méthoxybenzyl) (méthyl) amino.

**7.** Composition pharmaceutique comprenant un composé de formule I ;

ou le N-oxyde de l'azote de pipéridino dans $Q^1$;

ou un sel pharmaceutiquement acceptable de celui-ci ;

ou un sel d'ammonium quaternaire de celui-ci dans lequel l'azote de pipéridino dans $Q^1$ est un azote d'ammonium quadricovalent dans lequel le quatrième radical sur l'azote $R^1$ est un alkyle en $C_{1-4}$ ou un benzyle, et le contre-ion A associé est un anion pharmaceutiquement acceptable ; comme défini dans l'une quelconque des revendications 1 à 6 ; et un diluant ou support pharmaceutiquement acceptable.

**8.** Procédé d'élaboration d'un composé de formule I ; ou du N-oxyde de l'azote de pipéridino dans $Q^1$ ; ou d'un sel pharmaceutiquement acceptable de celui-ci ; ou d'un sel d'ammonium quaternaire de celui-ci dans lequel l'azote de pipéridino dans $Q^1$ est un azote d'ammonium quadricovalent dans lequel le quatrième radical sur l'azote $R^1$ est un alkyle en $C_{1-4}$ ou un benzyle, et le contre-ion A associé est un anion pharmaceutiquement acceptable ;

comme défini dans l'une quelconque des revendications 1 à 6 ; qui est caractérisé par :

(a) l'acylation d'une amine de formule $-NR^3R^4$ avec un ester de formule IV :

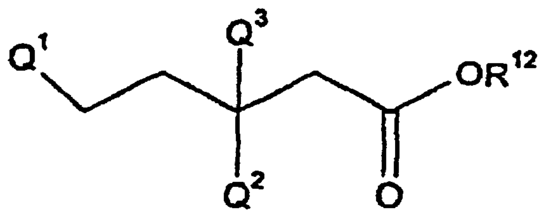

IV

dans laquelle $R^{12}$ est un radical alkyle approprié tel que, par exemple, un alkyle en $C_{1-3}$ ;

(b) pour un sel d'addition à un acide d'un composé de formule I, le traitement d'un composé correspondant de formule I qui est sous la forme de base libre, par un acide

(c) l'alkylation d'une amine de formule $Q^1$-H avec un aldéhyde de formule V :

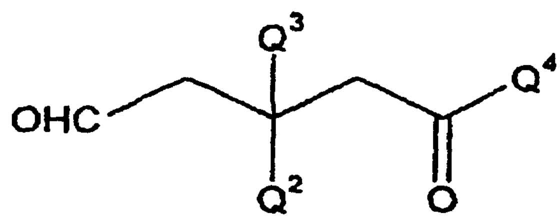

V

par alkylation réductive ;

(d) l'acylation d'une amine de formule $-NR^3R^4$ avec un acide de formule IV

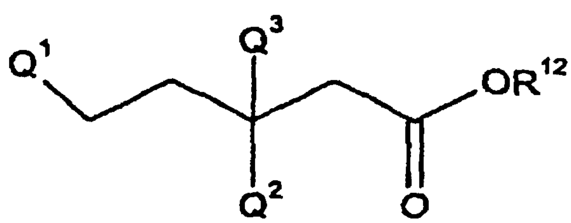

IV

dans laquelle $R^{12}$ est un hydrogène ;

(e) l'alkylation d'une amine de formule $Q^1$-H avec un agent d'alkylation de formule VI :

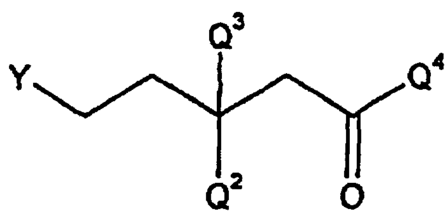

VI

dans laquelle Y est un groupe partant traditionnel ;

(f) pour un N-oxyde de l'azote de pipéridino dans $Q^1$, l'oxydation de l'azote d'un composé correspondant de formule I en employant un mode opératoire traditionnel ;

(g) pour un sel d'ammonium quaternaire de l'azote de pipéridino dans $Q^1$, l'alkylation de l'azote dans un composé correspondant de formule I avec un agent d'alkylation de formule $R^1Y$ dans laquelle Y est un groupe partant ;
(j) pour un composé de formule I renfermant un groupe hydroxy aromatique, le clivage de l'éther d'un composé correspondant de formule I renfermant un groupe alcoxy aromatique.